(19) Europäisches Patentamt
European Patent Office
Office européen des brevets

(11) EP 2 329 272 B1

(12) # FASCICULE DE BREVET EUROPEEN

(45) Date de publication et mention de la délivrance du brevet:
**09.10.2013 Bulletin 2013/41**

(21) Numéro de dépôt: **09740420.6**

(22) Date de dépôt: **30.07.2009**

(51) Int Cl.:
***G01N 33/566*** *(2006.01)* ***G01N 33/58*** *(2006.01)*

(86) Numéro de dépôt international:
**PCT/FR2009/051538**

(87) Numéro de publication internationale:
**WO 2010/012962 (04.02.2010 Gazette 2010/05)**

(54) **METHODE DE DETECTION DE L'INTERNALISATION DE PROTEINES MEMBRANAIRES**

VERFAHREN ZUM NACHWEIS VON MEMBRANPROTEININTERNALISIERUNG

METHOD FOR DETECTING MEMBRANE PROTEIN INTERNALIZATION

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **31.07.2008 FR 0855296**

(43) Date de publication de la demande:
**08.06.2011 Bulletin 2011/23**

(73) Titulaire: **Ion Beam Applications**
**1348 Louvain-la-Neuve (BE)**

(72) Inventeurs:
- **ZWIER, Jurriaan**
**30650 Rochefort Du Gard (FR)**
- **POOLE, Robert**
**Ringwood**
**Hampshire BH24 1PJ (GB)**
- **ANSANAY, Hervé**
**34000 Montpellier (FR)**
- **FINK, Michel**
**30200 Bagnols Sur Ceze (FR)**
- **TRINQUET, Eric**
**30130 Pont Saint Esprit (FR)**

(74) Mandataire: **Nevant, Marc**
**Cabinet Beau de Loménie**
**158, rue de l'Université**
**75340 Paris Cedex 07 (FR)**

(56) Documents cités:
**WO-A-2008/103393 US-A1- 2005 026 234**

- **DHONUKSHE P ET AL.: "Clathrin-Mediated Constitutive Endocytosis of PIN Auxin Efflux Carriers in Arabidopsis" CURRENT BIOLOGY, CURRENT SCIENCE, vol. 17, no. 6, 19 mars 2007 (2007-03-19), pages 520-527, XP005924454 GB doi:10.1016/j.cub.2007.01.052 ISSN: 0960-9822**
- **REAVES B J ET AL.: "The effect of wortmannin on the localisation of lysosomal type I integral membrane glycoproteins suggests a role for phosphoinositide 3-kinase activity in regulating membrane traffic late in the endocytic pathway" JOURNAL OF CELL SCIENCE, vol. 109, no. 4, 1996, pages 749-762, XP009114132 ISSN: 0021-9533**
- **ROOT D D: "In situ molecular association of dystrophin with actin revealed by sensitized emission immuno-resonance energy transfer" PROCEEDINGS OF THE NATIONAL ACADEMY OF SCIENCES OF THE UNITED STATES OF AMERICA, vol. 94, no. 11, 27 mai 1997 (1997-05-27), pages 5685-5690, XP000882831 US doi:10.1073/pnas.94.11.5685 ISSN: 0027-8424**
- **MAUREL DAMIEN ET AL: "Cell surface detection of membrane protein interaction with homogeneous time-resolved fluorescence resonance energy transfer technology." ANALYTICAL BIOCHEMISTRY, vol. 329, no. 2, 15 juin 2004 (2004-06-15) , pages 253-262, XP4509821 US ISSN: 0003-2697 DOI: 10.1016/j.ab. 2004.02.013**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 329 272 B1

LISTAGE DES SEQUENCES

<110> CIS BIO INTERNATIONAL
ZWIER, Jurriaan
POOLE, Robert
ANSANAY, Hervé
FINK, Michel
TRINQUET, Eric

<120> Méthode de détection de l'internalisation de protéines membranaires

<130> 1H189130 0029 WO PCT

<150> FR08/55296
<151> 2008-07-31

<160> 7

<170> PatentIn version 3.3

<210> 1
<211> 6
<212> PRT
<213> Séquence artificielle

<220>
<221> MISC_ FEATURE
<222> (3) .. (4)
<223> Xaa est un acide aminé quelconque

<400> 1

**Cys Cys Xaa Xaa Cys Cys**
**1 5**

<210> 2
<211> 8
<212> PRT
<213> Séquence artificielle

<400> 2

**Asp Tyr Lys Asp Asp Asp Asp Lys**
**1 5**

<210> 3
<211> 9
<212> PRT
<213> Séquence artificielle

<400> 3

**Tyr Pro Tyr Asp Val Pro Phe Tyr Ala**
**1 5**

<210> 4
<211> 7154
<212> DNA
<213> Séquence artificielle

<220>
<221> CMV_ promoter
<222> (232) .. (819)

<220>
<221> T8 sig_ peptide
<222> (923) .. (986)

<220>

<221> Gène codant pour l'épitope OFLAG _ CDS
<222> (1004) .. (1027)

<220>
<221> Gène codant pour l'enzyme Snap Tag _CDS
<222> (1046) .. (1591)

<220>
<221> Gène codant pour le récepteur V2 _ CDS
<222> (1598) .. (2710)

<220>
<221> BGH _ polyA_ signal
<222> (2754) .. (2978)

<220>
<221> Gène de résistance à la néomycine _ CDS
<222> (3862) .. (4656)

<220>
<221> Gène de résistance à l'ampicilline _ CDS
<222> (6158) .. (7018)

<400> 4

```
gacggatcgg gagatctccc gatcccctat ggtgcactct cagtacaatc tgctctgatg      60
ccgcatagtt aagccagtat ctgctccctg cttgtgtgtt ggaggtcgct gagtagtgcg     120
cgagcaaaat ttaagctaca acaaggcaag gcttgaccga caattgcatg aagaatctgc     180
ttagggttag gcgttttgcg ctgcttcgcg atgtacgggc cagatatacg cgttgacatt     240
gattattgac tagttattaa tagtaatcaa ttacggggtc attagttcat agcccatata     300
tggagttccg cgttacataa cttacggtaa atggcccgcc tggctgaccg cccaacgacc     360
cccgcccatt gacgtcaata atgacgtatg ttcccatagt aacgccaata gggactttcc     420
attgacgtca atgggtggag tatttacggt aaactgccca cttggcagta catcaagtgt     480
atcatatgcc aagtacgccc cctattgacg tcaatgacgg taaatggccc gcctggcatt     540
atgcccagta catgacctta tgggactttc ctacttggca gtacatctac gtattagtca     600
tcgctattac catggtgatg cggttttggc agtacatcaa tgggcgtgga tagcggtttg     660
actcacgggg atttccaagt ctccacccca ttgacgtcaa tgggagtttg ttttggcacc     720
aaaatcaacg ggactttcca aaatgtcgta acaactccgc cccattgacg caaatgggcg     780
gtaggcgtgt acggtgggag gtctatataa gcagagctct ctggctaact agagaaccca     840
ctgcttactg gcttatcgaa attaatacga ctcactatag ggagacccaa gctggctagc     900
gtttaaactt aagctttgag acatggcctt accagtgacc gccttgctcc tgccgctggc     960
```

cttgctgctc cacgccgcca ggccggccgc cgctagcggc atcgactaca aggacgacga 1020
tgacaaggcc ggcatcgatg ccatcatgga caaagactgc gaaatgaagc gcaccaccct 1080
ggatagccct ctgggcaagc tggaactgtc tgggtgcgaa cagggcctgc acgagatcaa 1140
gctgctgggc aaaggaacat ctgccgccga cgccgtggaa gtgcctgccc cagccgccgt 1200
gctgggcgga ccagagccac tgatgcaggc caccgcctgg ctcaacgcct actttcacca 1260
gcctgaggcc atcgaggagt tccctgtgcc agccctgcac cacccagtgt tccagcagga 1320
gagctttacc cgccaggtgc tgtggaaact gctgaaagtg gtgaagttcg gagaggtcat 1380
cagctaccag cagctggccg ccctggccgg caatcccgcc gccaccgccg ccgtgaaaac 1440
cgccctgagc ggaaatcccg tgcccattct gatcccctgc caccgggtgg tgtctagctc 1500
tggcgccgtg gggggctacg agggcgggct cgccgtgaaa gagtggctgc tggcccacga 1560
gggccacaga ctgggcaagc ctgggctggg tgatatcctc atggcgtcca ccacttccgc 1620
tgtgcctggg catccctctc tgcccagcct gcccagcaac agcagccagg agaggccact 1680
ggacacccgg gacccgctgc tagcccgggc ggagctggcg ctgctctcca tagtctttgt 1740
ggctgtggcc ctgagcaatg gcctggtgct ggcggcccta gctcggcggg gccggcgggg 1800
ccactgggca cccatacacg tcttcattgg ccacttgtgc ctggccgacc tggccgtggc 1860
tctgttccaa gtgctgcccc agctggcctg gaaggccacc gaccgcttcc gtgggccaga 1920
tgccctgtgt cgggccgtga agtatctgca gatggtgggc atgtatgcct cctcctacat 1980
gatcctggcc atgacgctgg accgccaccg tgccatctgc cgtcccatgc tggcgtaccg 2040
ccatggaagt ggggctcact ggaaccggcc ggtgctagtg gcttgggcct tctcgctcct 2100
tctcagcctg ccccagctct tcatcttcgc ccagcgcaac gtggaaggtg gcagcggggt 2160
cactgactgc tgggcctgct ttgcggagcc ctggggccgt cgcacctatg tcacctggat 2220
tgccctgatg gtgttcgtgg cacctaccct gggtatcgcc gcctgccagg tgctcatctt 2280
ccgggagatt catgccagtc tggtgccagg gccatcagag aggcctgggg ggcgccgcag 2340
gggacgccgg acaggcagcc ccggtgaggg agcccacgtg tcagcagctg tggccaagac 2400
tgtgaggatg acgctagtga ttgtggtcgt ctatgtgctg tgctgggcac ccttcttcct 2460
ggtgcagctg tgggccgcgt gggacccgga ggcacctctg gaaggggcgc cctttgtgct 2520
actcatgttg ctggccagcc tcaacagctg caccaacccc tggatctatg catctttcag 2580
cagcagcgtg tcctcagagc tgcgaagctt gctctgctgt gcccggggac gcaccccacc 2640
cagcctgggt ccccaagatg agtcctgcac caccgccagc tcctccctgg ccaaggacac 2700
ttcatcgtga ctcgagtcta gagggcccgt ttaaacccgc tgatcagcct cgactgtgcc 2760
ttctagttgc cagccatctg ttgtttgccc ctcccccgtg ccttccttga ccctggaagg 2820
tgccactccc actgtccttt cctaataaaa tgaggaaatt gcatcgcatt gtctgagtag 2880

gtgtcattct attctggggg gtggggtggg gcaggacagc aagggggagg attgggaaga 2940
caatagcagg catgctgggg atgcggtggg ctctatggct tctgaggcgg aaagaaccag 3000
ctggggctct agggggtatc cccacgcgcc ctgtagcggc gcattaagcg cggcgggtgt 3060
ggtggttacg cgcagcgtga ccgctacact tgccagcgcc ctagcgcccg ctcctttcgc 3120
tttcttccct tcctttctcg ccacgttcgc cggctttccc cgtcaagctc taaatcgggg 3180
gctcccttta gggttccgat ttagtgcttt acggcacctc gaccccaaaa aacttgatta 3240
gggtgatggt tcacgtagtg ggccatcgcc ctgatagacg gtttttcgcc ctttgacgtt 3300
ggagtccacg ttctttaata gtggactctt gttccaaact ggaacaacac tcaaccctat 3360
ctcggtctat tcttttgatt tataagggat tttgccgatt tcggcctatt ggttaaaaaa 3420
tgagctgatt taacaaaaat ttaacgcgaa ttaattctgt ggaatgtgtg tcagttaggg 3480
tgtggaaagt ccccaggctc cccagcaggc agaagtatgc aaagcatgca tctcaattag 3540
tcagcaacca ggtgtggaaa gtccccaggc tccccagcag gcagaagtat gcaaagcatg 3600
catctcaatt agtcagcaac catagtcccg cccctaactc cgcccatccc gcccctaact 3660
ccgcccagtt ccgcccattc tccgccccat ggctgactaa ttttttttat ttatgcagag 3720
gccgaggccg cctctgcctc tgagctattc cagaagtagt gaggaggctt ttttggaggc 3780
ctaggctttt gcaaaaagct cccgggagct tgtatatcca ttttcggatc tgatcaagag 3840
acaggatgag gatcgtttcg catgattgaa caagatggat tgcacgcagg ttctccggcc 3900
gcttgggtgg agaggctatt cggctatgac tgggcacaac agacaatcgg ctgctctgat 3960
gccgccgtgt tccggctgtc agcgcagggg cgcccggttc tttttgtcaa gaccgacctg 4020
tccggtgccc tgaatgaact gcaggacgag gcagcgcggc tatcgtggct ggccacgacg 4080
ggcgttcctt gcgcagctgt gctcgacgtt gtcactgaag cgggaaggga ctggctgcta 4140
ttgggcgaag tgccggggca ggatctcctg tcatctcacc ttgctcctgc cgagaaagta 4200
tccatcatgg ctgatgcaat gcggcggctg catacgcttg atccggctac ctgcccattc 4260
gaccaccaag cgaaacatcg catcgagcga gcacgtactc ggatggaagc cggtcttgtc 4320
gatcaggatg atctggacga agagcatcag gggctcgcgc cagccgaact gttcgccagg 4380
ctcaaggcgc gcatgcccga cggcgaggat ctcgtcgtga cccatggcga tgcctgcttg 4440
ccgaatatca tggtggaaaa tggccgcttt tctggattca tcgactgtgg ccggctgggt 4500
gtggcggacc gctatcagga catagcgttg gctacccgtg atattgctga agagcttggc 4560
ggcgaatggg ctgaccgctt cctcgtgctt tacggtatcg ccgctcccga ttcgcagcgc 4620
atcgccttct atcgccttct tgacgagttc ttctgagcgg gactctgggg ttcgaaatga 4680
ccgaccaagc gacgcccaac ctgccatcac gagatttcga ttccaccgcc gccttctatg 4740
aaaggttggg cttcggaatc gttttccggg acgccggctg gatgatcctc cagcgcgggg 4800
atctcatgct ggagttcttc gcccacccca acttgtttat tgcagcttat aatggttaca 4860

aataaagcaa tagcatcaca aatttcacaa ataaagcatt tttttcactg cattctagtt 4920
gtggtttgtc caaactcatc aatgtatctt atcatgtctg tataccgtcg acctctagct 4980
agagcttggc gtaatcatgg tcatagctgt ttcctgtgtg aaattgttat ccgctcacaa 5040
ttccacacaa catacgagcc ggaagcataa agtgtaaagc ctggggtgcc taatgagtga 5100
gctaactcac attaattgcg ttgcgctcac tgcccgcttt ccagtcggga aacctgtcgt 5160
gccagctgca ttaatgaatc ggccaacgcg cggggagagg cggtttgcgt attgggcgct 5220
cttccgcttc ctcgctcact gactcgctgc gctcggtcgt tcggctgcgg cgagcggtat 5280
cagctcactc aaaggcggta atacggttat ccacagaatc aggggataac gcaggaaaga 5340
acatgtgagc aaaaggccag caaaaggcca ggaaccgtaa aaaggccgcg ttgctggcgt 5400
ttttccatag gctccgcccc cctgacgagc atcacaaaaa tcgacgctca agtcagaggt 5460
ggcgaaaccc gacaggacta taaagatacc aggcgtttcc ccctggaagc tccctcgtgc 5520
gctctcctgt tccgaccctg ccgcttaccg gatacctgtc cgcctttctc ccttcgggaa 5580
gcgtggcgct ttctcatagc tcacgctgta ggtatctcag ttcggtgtag gtcgttcgct 5640
ccaagctggg ctgtgtgcac gaaccccccg ttcagcccga ccgctgcgcc ttatccggta 5700
actatcgtct tgagtccaac ccggtaagac acgacttatc gccactggca gcagccactg 5760
gtaacaggat tagcagagcg aggtatgtag gcggtgctac agagttcttg aagtggtggc 5820
ctaactacgg ctacactaga agaacagtat ttggtatctg cgctctgctg aagccagtta 5880
ccttcggaaa aagagttggt agctcttgat ccggcaaaca aaccaccgct ggtagcggtt 5940
tttttgtttg caagcagcag attacgcgca gaaaaaaagg atctcaagaa gatcctttga 6000
tcttttctac ggggtctgac gctcagtgga acgaaaactc acgttaaggg attttggtca 6060
tgagattatc aaaaaggatc ttcacctaga tccttttaaa ttaaaaatga agttttaaat 6120
caatctaaag tatatatgag taaacttggt ctgacagtta ccaatgctta atcagtgagg 6180
cacctatctc agcgatctgt ctatttcgtt catccatagt tgcctgactc cccgtcgtgt 6240
agataactac gatacgggag ggcttaccat ctggccccag tgctgcaatg ataccgcgag 6300
acccacgctc accggctcca gatttatcag caataaacca gccagccgga agggccgagc 6360
gcagaagtgg tcctgcaact ttatccgcct ccatccagtc tattaattgt tgccgggaag 6420
ctagagtaag tagttcgcca gttaatagtt tgcgcaacgt tgttgccatt gctacaggca 6480
tcgtggtgtc acgctcgtcg tttggtatgg cttcattcag ctccggttcc caacgatcaa 6540
ggcgagttac atgatccccc atgttgtgca aaaaagcggt tagctccttc ggtcctccga 6600
tcgttgtcag aagtaagttg gccgcagtgt tatcactcat ggttatggca gcactgcata 6660
attctcttac tgtcatgcca tccgtaagat gcttttctgt gactggtgag tactcaacca 6720
agtcattctg agaatagtgt atgcggcgac cgagttgctc ttgcccggcg tcaatacggg 6780

```
ataataccgc gccacatagc agaactttaa aagtgctcat cattggaaaa cgttcttcgg   6840
ggcgaaaact ctcaaggatc ttaccgctgt tgagatccag ttcgatgtaa cccactcgtg   6900
cacccaactg atcttcagca tcttttactt tcaccagcgt ttctgggtga gcaaaaacag   6960
gaaggcaaaa tgccgcaaaa aagggaataa gggcgacacg gaaatgttga atactcatac   7020
tcttcctttt tcaatattat tgaagcattt atcagggtta ttgtctcatg agcggataca   7080
tatttgaatg tatttagaaa aataaacaaa taggggttcc gcgcacattt ccccgaaaag   7140
tgccacctga cgtc                                                     7154
```

<210> 5
<211> 6773
<212> DNA
<213> Séquence artificielle

<220>
<221> BGH _ polyA_ signal
<222> (82) .. (306)

<220>
<221> Gène de résistance à la néomycine _ CDS
<222> (1190) .. (1984)

<220>
<221> Gène de résistance à l’ampiciline _ CDS
<222> (3486) .. (4346)

<220>
<221> CMV _ promoter
<222> (4714) .. (5301)

<220>
<221> Gène codant pour l’épitope FLAG _ CDS
<222> (5447) .. (5476)

<220>
<221> Gène codant pour la béta- arrestine 1 humaine _ CDS
<222> (5507) .. (6760)

<400> 5

cttgtacaaa gtggtgatat ccagcacagt ggcggccgct cgagtctaga gggcccgttt 60

aaacccgctg atcagcctcg actgtgcctt ctagttgcca gccatctgtt gtttgcccct 120

cccccgtgcc ttccttgacc ctggaaggtg ccactcccac tgtcctttcc taataaaatg 180

aggaaattgc atcgcattgt ctgagtaggt gtcattctat tctggggggt ggggtggggc 240

aggacagcaa gggggaggat tgggaagaca atagcaggca tgctggggat gcggtgggct 300

ctatggcttc tgaggcggaa agaaccagct ggggctctag ggggtatccc cacgcgccct 360

gtagcggcgc attaagcgcg gcgggtgtgg tggttacgcg cagcgtgacc gctacacttg 420

ccagcgccct agcgcccgct cctttcgctt tcttcccttc ctttctcgcc acgttcgccg 480

gctttccccg tcaagctcta aatcgggggc tccctttagg gttccgattt agtgctttac 540

ggcacctcga ccccaaaaaa cttgattagg gtgatggttc acgtagtggg ccatcgccct 600

gatagacggt ttttcgccct ttgacgttgg agtccacgtt ctttaatagt ggactcttgt 660
tccaaactgg aacaacactc aaccctatct cggtctattc ttttgattta taagggattt 720
tgccgatttc ggcctattgg ttaaaaaatg agctgattta acaaaaattt aacgcgaatt 780
aattctgtgg aatgtgtgtc agttagggtg tggaaagtcc ccaggctccc cagcaggcag 840
aagtatgcaa agcatgcatc tcaattagtc agcaaccagg tgtggaaagt ccccaggctc 900
cccagcaggc agaagtatgc aaagcatgca tctcaattag tcagcaacca tagtcccgcc 960
cctaactccg cccatcccgc ccctaactcc gcccagttcc gcccattctc cgccccatgg 1020
ctgactaatt ttttttattt atgcagaggc cgaggccgcc tctgcctctg agctattcca 1080
gaagtagtga ggaggctttt ttggaggcct aggcttttgc aaaaagctcc cgggagcttg 1140
tatatccatt ttcggatctg atcaagagac aggatgagga tcgtttcgca tgattgaaca 1200
agatggattg cacgcaggtt ctccggccgc ttgggtggag aggctattcg gctatgactg 1260
ggcacaacag acaatcggct gctctgatgc cgccgtgttc cggctgtcag cgcaggggcg 1320
cccggttctt tttgtcaaga ccgacctgtc cggtgccctg aatgaactgc aggacgaggc 1380
agcgcggcta tcgtggctgg ccacgacggg cgttccttgc gcagctgtgc tcgacgttgt 1440
cactgaagcg ggaagggact ggctgctatt gggcgaagtg ccggggcagg atctcctgtc 1500
atctcacctt gctcctgccg agaaagtatc catcatggct gatgcaatgc ggcggctgca 1560
tacgcttgat ccggctacct gcccattcga ccaccaagcg aaacatcgca tcgagcgagc 1620
acgtactcgg atggaagccg gtcttgtcga tcaggatgat ctggacgaag agcatcaggg 1680
gctcgcgcca gccgaactgt tcgccaggct caaggcgcgc atgcccgacg gcgaggatct 1740
cgtcgtgacc catggcgatg cctgcttgcc gaatatcatg gtggaaaatg gccgcttttc 1800
tggattcatc gactgtggcc ggctgggtgt ggcggaccgc tatcaggaca tagcgttggc 1860
tacccgtgat attgctgaag agcttggcgg cgaatgggct gaccgcttcc tcgtgcttta 1920
cggtatcgcc gctcccgatt cgcagcgcat cgccttctat cgccttcttg acgagttctt 1980
ctgagcggga ctctggggtt cgaaatgacc gaccaagcga cgcccaacct gccatcacga 2040
gatttcgatt ccaccgccgc cttctatgaa aggttgggct tcggaatcgt tttccgggac 2100
gccggctgga tgatcctcca gcgcggggat ctcatgctgg agttcttcgc ccaccccaac 2160
ttgtttattg cagcttataa tggttacaaa taaagcaata gcatcacaaa tttcacaaat 2220
aaagcatttt tttcactgca ttctagttgt ggtttgtcca aactcatcaa tgtatcttat 2280
catgtctgta taccgtcgac ctctagctag agcttggcgt aatcatggtc atagctgttt 2340
cctgtgtgaa attgttatcc gctcacaatt ccacacaaca tacgagccgg aagcataaag 2400
tgtaaagcct ggggtgccta atgagtgagc taactcacat taattgcgtt gcgctcactg 2460
cccgctttcc agtcgggaaa cctgtcgtgc cagctgcatt aatgaatcgg ccaacgcgcg 2520

gggagaggcg gtttgcgtat tgggcgctct tccgcttcct cgctcactga ctcgctgcgc 2580

tcggtcgttc ggctgcggcg agcggtatca gctcactcaa aggcggtaat acggttatcc 2640

acagaatcag gggataacgc aggaaagaac atgtgagcaa aaggccagca aaaggccagg 2700

aaccgtaaaa aggccgcgtt gctggcgttt ttccataggc tccgcccccc tgacgagcat 2760

cacaaaaatc gacgctcaag tcagaggtgg cgaaacccga caggactata aagataccag 2820

gcgtttcccc ctggaagctc cctcgtgcgc tctcctgttc cgaccctgcc gcttaccgga 2880

tacctgtccg cctttctccc ttcgggaagc gtggcgcttt ctcatagctc acgctgtagg 2940

tatctcagtt cggtgtaggt cgttcgctcc aagctgggct gtgtgcacga accccccgtt 3000

cagcccgacc gctgcgcctt atccggtaac tatcgtcttg agtccaaccc ggtaagacac 3060

gacttatcgc cactggcagc agccactggt aacaggatta gcagagcgag gtatgtaggc 3120

ggtgctacag agttcttgaa gtggtggcct aactacggct acactagaag aacagtattt 3180

ggtatctgcg ctctgctgaa gccagttacc ttcggaaaaa gagttggtag ctcttgatcc 3240

ggcaaacaaa ccaccgctgg tagcggtttt tttgtttgca agcagcagat tacgcgcaga 3300

aaaaaaggat ctcaagaaga tcctttgatc ttttctacgg ggtctgacgc tcagtggaac 3360

gaaaactcac gttaagggat tttggtcatg agattatcaa aaaggatctt cacctagatc 3420

cttttaaatt aaaaatgaag ttttaaatca atctaaagta tatatgagta aacttggtct 3480

gacagttacc aatgcttaat cagtgaggca cctatctcag cgatctgtct atttcgttca 3540

tccatagttg cctgactccc cgtcgtgtag ataactacga tacgggaggg cttaccatct 3600

ggccccagtg ctgcaatgat accgcgagac ccacgctcac cggctccaga tttatcagca 3660

ataaaccagc cagccggaag ggccgagcgc agaagtggtc ctgcaacttt atccgcctcc 3720

atccagtcta ttaattgttg ccgggaagct agagtaagta gttcgccagt taatagtttg 3780

cgcaacgttg ttgccattgc tacaggcatc gtggtgtcac gctcgtcgtt tggtatggct 3840

tcattcagct ccggttccca acgatcaagg cgagttacat gatcccccat gttgtgcaaa 3900

aaagcggtta gctccttcgg tcctccgatc gttgtcagaa gtaagttggc cgcagtgtta 3960

tcactcatgg ttatggcagc actgcataat tctcttactg tcatgccatc cgtaagatgc 4020

ttttctgtga ctggtgagta ctcaaccaag tcattctgag aatagtgtat gcggcgaccg 4080

agttgctctt gcccggcgtc aatacgggat aataccgcgc cacatagcag aactttaaaa 4140

gtgctcatca ttggaaaacg ttcttcgggg cgaaaactct caaggatctt accgctgttg 4200

agatccagtt cgatgtaacc cactcgtgca cccaactgat cttcagcatc ttttactttc 4260

accagcgttt ctgggtgagc aaaaacagga aggcaaaatg ccgcaaaaaa gggaataagg 4320

gcgacacgga aatgttgaat actcatactc ttcctttttc aatattattg aagcatttat 4380

cagggttatt gtctcatgag cggatacata tttgaatgta tttagaaaaa taaacaaata 4440

ggggttccgc gcacatttcc ccgaaaagtg ccacctgacg tcgacggatc gggagatctc 4500

ccgatcccct atggtgcact ctcagtacaa tctgctctga tgccgcatag ttaagccagt 4560
atctgctccc tgcttgtgtg ttggaggtcg ctgagtagtg cgcgagcaaa atttaagcta 4620
caacaaggca aggcttgacc gacaattgca tgaagaatct gcttagggtt aggcgttttg 4680
cgctgcttcg cgatgtacgg gccagatata cgcgttgaca ttgattattg actagttatt 4740
aatagtaatc aattacgggg tcattagttc atagcccata tatggagttc cgcgttacat 4800
aacttacggt aaatggcccg cctggctgac cgcccaacga cccccgccca ttgacgtcaa 4860
taatgacgta tgttcccata gtaacgccaa tagggacttt ccattgacgt caatgggtgg 4920
agtatttacg gtaaactgcc cacttggcag tacatcaagt gtatcatatg ccaagtacgc 4980
cccctattga cgtcaatgac ggtaaatggc ccgcctggca ttatgcccag tacatgacct 5040
tatgggactt tcctacttgg cagtacatct acgtattagt catcgctatt accatggtga 5100
tgcggttttg gcagtacatc aatgggcgtg gatagcggtt tgactcacgg ggatttccaa 5160
gtctccaccc cattgacgtc aatgggagtt tgttttggca ccaaaatcaa cgggactttc 5220
caaaatgtcg taacaactcc gccccattga cgcaaatggg cggtaggcgt gtacggtggg 5280
aggtctatat aagcagagct ctctggctaa ctagagaacc cactgcttac tggcttatcg 5340
aaattaatac gactcactat agggagaccc aagctggcta gcgtttaaac ttaagcttgg 5400
taccgagctc ggatccacta gtccagtgtg gtggaattct gcagatatgg actacaagga 5460
cgacgacgac aaggatatca caagtttgta caaaaaagca ggcaccatgg gcgacaaagg 5520
gacccgagtg ttcaagaagg ccagtccaaa tggaaagctc accgtctacc tgggaaagcg 5580
ggactttgtg gaccacatcg acctcgtgga ccctgtggat ggtgtggtcc tggtggatcc 5640
tgagtatctc aaagagcgga gagtctatgt gacgctgacc tgcgccttcc gctatggccg 5700
ggaggacctg gatgtcctgg gcctgacctt tcgcaaggac ctgtttgtgg ccaacgtaca 5760
gtcgttccca ccggcccccg aggacaagaa gcccctgacg cggctgcagg aacgcctcat 5820
caagaagctg ggcgagcacg cttacccttt cacctttgag atccctccaa accttccatg 5880
ttctgtgaca ctgcagccgg ggcccgaaga cacggggaag gcttgcggtg tggactatga 5940
agtcaaagcc ttctgcgcgg agaatttgga ggagaagatc cacaagcgga attctgtgcg 6000
tctggtcatc cggaaggttc agtatgcccc agagaggcct ggcccccagc ccacagccga 6060
gaccaccagg cagttcctca tgtcggacaa gcccttgcac ctagaagcct ctctggataa 6120
ggagatctat taccatggag aacccatcag cgtcaacgtc cacgtcacca acaacaccaa 6180
caagacggtg aagaagatca agatctcagt gcgccagtat gcagacatct gccttttcaa 6240
cacagctcag tacaagtgcc ctgttgccat ggaagaggct gatgacactg tggcacccag 6300
ctcgacgttc tgcaaggtct acacactgac cccccttccta gccaataacc gagagaagcg 6360
gggcctcgcc ttggacggga agctcaagca cgaagacacg aacttggcct ctagcacccct 6420

gttgagggaa ggtgccaacc gtgagatcct ggggatcatt gtttcctaca aagtgaaagt 6480

gaagctggtg gtgtctcggg gcggcctgtt gggagatctt gcatccagcg acgtggccgt 6540

ggaactgccc ttcaccctaa tgcaccccaa gcccaaagag gaacccccgc atcgggaagt 6600

tccagagaac gagacgccag tagataccaa tctcatagaa cttgacacaa atgatgacga 6660

cattgtattt gaggactttg ctcgccagag actgaaaggc atgaaggatg acaaggagga 6720

agaggaggat ggtaccggct ctccacagct caacaacaga tagacccagc ttt 6773

<210> 6
<211> 7130
<212> DNA
<213> Séquence artificielle

<220>
<221> CMV _ promoter
<222> (232) .. (819)

<220>
<221> T8 _ sig_ peptide
<222> (923) .. (986)

<220>
<221> Gène codant pour l'épitope FLAG _ CDS
<222> (1004) .. (1027)

<220>
<221> Gène codant pour l'enzyme Snap Tag _ CDS
<222> (1046) .. (1591)

<220>
<221> Gène codant pour le récepteur CXCR7 _ CDS
<222> (1595) .. (2687)

<220>
<221> BGH _ polyA_ signal
<222> (2730) .. (2954)

<220>
<221> Gène de résistance à la néomycine _ CDS
<222> (3838) .. (4632)

<220>
<221> Gène de résistance à l'ampicilline _ CDS
<222> (6134) .. (6994)

<400> 6

gacggatcgg gagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgttgacatt 240

gattattgac tagttattaa tagtaatcaa ttacggggtc attagttcat agcccatata 300

tggagttccg cgttacataa cttacggtaa atggcccgcc tggctgaccg cccaacgacc 360

cccgcccatt gacgtcaata atgacgtatg ttcccatagt aacgccaata gggactttcc 420

attgacgtca atgggtggag tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagctttgag acatggcctt accagtgacc gccttgctcc tgccgctggc 960
cttgctgctc cacgccgcca ggccggccgc cgctagcggc atcgactaca aggacgacga 1020
tgacaaggcc ggcatcgatg ccatcatgga caaagactgc gaaatgaagc gcaccaccct 1080
ggatagccct ctgggcaagc tggaactgtc tgggtgcgaa cagggcctgc acgagatcaa 1140
gctgctgggc aaaggaacat ctgccgccga cgccgtggaa gtgcctgccc cagccgccgt 1200
gctgggcgga ccagagccac tgatgcaggc caccgcctgg ctcaacgcct actttcacca 1260
gcctgaggcc atcgaggagt tccctgtgcc agccctgcac cacccagtgt tccagcagga 1320
gagctttacc cgccaggtgc tgtggaaact gctgaaagtg gtgaagttcg gagaggtcat 1380
cagctaccag cagctggccg ccctggccgg caatcccgcc gccaccgccg ccgtgaaaac 1440
cgccctgagc ggaaatcccg tgcccattct gatcccctgc caccgggtgg tgtctagctc 1500
tggcgccgtg gggggctacg agggcgggct cgccgtgaaa gagtggctgc tggcccacga 1560
gggccacaga ctgggcaagc ctgggctggg tgatatcctg gatctgcatc tcttcgacta 1620
ctcagagcca gggaacttct cggacatcag ctggccatgc aacagcagcg actgcatcgt 1680
ggtggacacg gtgatgtgtc ccaacatgcc caacaaaagc gtcctgctct acacgctctc 1740
cttcatttac attttcatct tcgtcatcgg catgattgcc aactccgtgg tggtctgggt 1800
gaatatccag gccaagacca caggctatga cacgcactgc tacatcttga acctggccat 1860
tgccgacctg tgggttgtcc tcaccatccc agtctgggtg gtcagtctcg tgcagcacaa 1920
ccagtggccc atgggcgagc tcacgtgcaa agtcacacac ctcatcttct ccatcaacct 1980
cttcagcagc attttcttcc tcacgtgcat gagcgtggac cgctacctct ccatcaccta 2040
cttcaccaac acccccagca gcaggaagaa gatggtacgc cgtgtcgtct gcatcctggt 2100
gtggctgctg gccttctgcg tgtctctgcc tgacacctac tacctgaaga ccgtcacgtc 2160
tgcgtccaac aatgagacct actgccggtc cttctacccc gagcacagca tcaaggagtg 2220
gctgatcggc atggagctgg tctccgttgt cttgggcttt gccgttccct tctccattat 2280
cgctgtcttc tacttcctgc tggccagagc catctcggcg tccagtgacc aggagaagca 2340

cagcagccgg aagatcatct tctcctacgt ggtggtcttc cttgtctgct ggttgcccta 2400
ccacgtggcg gtgctgctgg acatcttctc catcctgcac tacatccctt tcacctgccg 2460
gctggagcac gccctcttca cggccctgca tgtcacacag tgcctgtcgc tggtgcactg 2520
ctgcgtcaac cctgtcctct acagcttcat caatcgcaac tacaggtacg agctgatgaa 2580
ggccttcatc ttcaagtact cggccaaaac agggctcacc aagctcatcg atgcctccag 2640
agtctcagag acggagtact ctgccttgga gcagagcacc aaatgactcg agtctagagg 2700
gcccgtttaa acccgctgat cagcctcgac tgtgccttct agttgccagc catctgttgt 2760
ttgcccctcc cccgtgcctt ccttgaccct ggaaggtgcc actcccactg tcctttccta 2820
ataaaatgag gaaattgcat cgcattgtct gagtaggtgt cattctattc tggggggtgg 2880
ggtggggcag gacagcaagg gggaggattg ggaagacaat agcaggcatg ctggggatgc 2940
ggtgggctct atggcttctg aggcggaaag aaccagctgg ggctctaggg ggtatcccca 3000
cgcgccctgt agcggcgcat taagcgcggc gggtgtggtg gttacgcgca gcgtgaccgc 3060
tacacttgcc agcgccctag cgcccgctcc tttcgctttc ttcccttcct ttctcgccac 3120
gttcgccggc tttccccgtc aagctctaaa tcgggggctc cctttagggt tccgatttag 3180
tgctttacgg cacctcgacc ccaaaaaact tgattagggt gatggttcac gtagtgggcc 3240
atcgccctga tagacggttt ttcgcccttt gacgttggag tccacgttct ttaatagtgg 3300
actcttgttc caaactggaa caacactcaa ccctatctcg gtctattctt ttgatttata 3360
agggattttg ccgatttcgg cctattggtt aaaaaatgag ctgatttaac aaaaatttaa 3420
cgcgaattaa ttctgtggaa tgtgtgtcag ttagggtgtg gaaagtcccc aggctcccca 3480
gcaggcagaa gtatgcaaag catgcatctc aattagtcag caaccaggtg tggaaagtcc 3540
ccaggctccc cagcaggcag aagtatgcaa agcatgcatc tcaattagtc agcaaccata 3600
gtcccgcccc taactccgcc catcccgccc ctaactccgc ccagttccgc ccattctccg 3660
ccccatggct gactaatttt ttttatttat gcagaggccg aggccgcctc tgcctctgag 3720
ctattccaga agtagtgagg aggctttttt ggaggcctag gcttttgcaa aaagctcccg 3780
ggagcttgta tatccatttt cggatctgat caagagacag gatgaggatc gtttcgcatg 3840
attgaacaag atggattgca cgcaggttct ccggccgctt gggtggagag gctattcggc 3900
tatgactggg cacaacagac aatcggctgc tctgatgccg ccgtgttccg gctgtcagcg 3960
caggggcgcc cggttctttt tgtcaagacc gacctgtccg gtgccctgaa tgaactgcag 4020
gacgaggcag cgcggctatc gtggctggcc acgacgggcg ttccttgcgc agctgtgctc 4080
gacgttgtca ctgaagcggg aagggactgg ctgctattgg gcgaagtgcc ggggcaggat 4140
ctcctgtcat ctcaccttgc tcctgccgag aaagtatcca tcatggctga tgcaatgcgg 4200
cggctgcata cgcttgatcc ggctacctgc ccattcgacc accaagcgaa acatcgcatc 4260
gagcgagcac gtactcggat ggaagccggt cttgtcgatc aggatgatct ggacgaagag 4320

catcaggggc tcgcgccagc cgaactgttc gccaggctca aggcgcgcat gcccgacggc 4380
gaggatctcg tcgtgaccca tggcgatgcc tgcttgccga atatcatggt ggaaaatggc 4440
cgcttttctg gattcatcga ctgtggccgg ctgggtgtgg cggaccgcta tcaggacata 4500
gcgttggcta cccgtgatat tgctgaagag cttggcggcg aatgggctga ccgcttcctc 4560
gtgctttacg gtatcgccgc tcccgattcg cagcgcatcg ccttctatcg ccttcttgac 4620
gagttcttct gagcgggact ctggggttcg aaatgaccga ccaagcgacg cccaacctgc 4680
catcacgaga tttcgattcc accgccgcct tctatgaaag gttgggcttc ggaatcgttt 4740
tccgggacgc cggctggatg atcctccagc gcggggatct catgctggag ttcttcgccc 4800
accccaactt gtttattgca gcttataatg gttacaaata aagcaatagc atcacaaatt 4860
tcacaaataa agcatttttt tcactgcatt ctagttgtgg tttgtccaaa ctcatcaatg 4920
tatcttatca tgtctgtata ccgtcgacct ctagctagag cttggcgtaa tcatggtcat 4980
agctgtttcc tgtgtgaaat tgttatccgc tcacaattcc acacaacata cgagccggaa 5040
gcataaagtg taaagcctgg ggtgcctaat gagtgagcta actcacatta attgcgttgc 5100
gctcactgcc cgctttccag tcgggaaacc tgtcgtgcca gctgcattaa tgaatcggcc 5160
aacgcgcggg gagaggcggt ttgcgtattg ggcgctcttc cgcttcctcg ctcactgact 5220
cgctgcgctc ggtcgttcgg ctgcggcgag cggtatcagc tcactcaaag gcggtaatac 5280
ggttatccac agaatcaggg gataacgcag gaaagaacat gtgagcaaaa ggccagcaaa 5340
aggccaggaa ccgtaaaaag gccgcgttgc tggcgttttt ccataggctc cgcccccctg 5400
acgagcatca caaaaatcga cgctcaagtc agaggtggcg aaacccgaca ggactataaa 5460
gataccaggc gtttccccct ggaagctccc tcgtgcgctc tcctgttccg accctgccgc 5520
ttaccggata cctgtccgcc tttctccctt cgggaagcgt ggcgctttct catagctcac 5580
gctgtaggta tctcagttcg gtgtaggtcg ttcgctccaa gctgggctgt gtgcacgaac 5640
cccccgttca gcccgaccgc tgcgccttat ccggtaacta tcgtcttgag tccaacccgg 5700
taagacacga cttatcgcca ctggcagcag ccactggtaa caggattagc agagcgaggt 5760
atgtaggcgg tgctacagag ttcttgaagt ggtggcctaa ctacggctac actagaagaa 5820
cagtatttgg tatctgcgct ctgctgaagc cagttacctt cggaaaaaga gttggtagct 5880
cttgatccgg caaacaaacc accgctggta gcggtttttt tgtttgcaag cagcagatta 5940
cgcgcagaaa aaaaggatct caagaagatc ctttgatctt ttctacgggg tctgacgctc 6000
agtggaacga aaactcacgt taagggattt tggtcatgag attatcaaaa aggatcttca 6060
cctagatcct tttaaattaa aaatgaagtt ttaaatcaat ctaaagtata tatgagtaaa 6120
cttggtctga cagttaccaa tgcttaatca gtgaggcacc tatctcagcg atctgtctat 6180
ttcgttcatc catagttgcc tgactccccg tcgtgtagat aactacgata cgggagggct 6240

taccatctgg ccccagtgct gcaatgatac cgcgagaccc acgctcaccg gctccagatt 6300

tatcagcaat aaaccagcca gccggaaggg ccgagcgcag aagtggtcct gcaactttat 6360

ccgcctccat ccagtctatt aattgttgcc gggaagctag agtaagtagt tcgccagtta 6420

atagtttgcg caacgttgtt gccattgcta caggcatcgt ggtgtcacgc tcgtcgtttg 6480

gtatggcttc attcagctcc ggttcccaac gatcaaggcg agttacatga tcccccatgt 6540

tgtgcaaaaa agcggttagc tccttcggtc ctccgatcgt tgtcagaagt aagttggccg 6600

cagtgttatc actcatggtt atggcagcac tgcataattc tcttactgtc atgccatccg 6660

taagatgctt ttctgtgact ggtgagtact caaccaagtc attctgagaa tagtgtatgc 6720

ggcgaccgag ttgctcttgc ccggcgtcaa tacgggataa taccgcgcca catagcagaa 6780

ctttaaaagt gctcatcatt ggaaaacgtt cttcggggcg aaaactctca aggatcttac 6840

cgctgttgag atccagttcg atgtaaccca ctcgtgcacc caactgatct tcagcatctt 6900

ttactttcac cagcgtttct gggtgagcaa aaacaggaag gcaaaatgcc gcaaaaaagg 6960

gaataagggc gacacggaaa tgttgaatac tcatactctt cctttttcaa tattattgaa 7020

gcatttatca gggttattgt ctcatgagcg gatacatatt tgaatgtatt tagaaaaata 7080

aacaaatagg ggttccgcgc acatttcccc gaaaagtgcc acctgacgtc 7130

<210> 7
<211> 7295
<212> DNA
<213> Séquence artificielle

<220>
<221> CMV _ promoter
<222> (232) .. (819)

<220>
<221> T8 _ sig_ peptide
<222> (923) .. (986)

<220>
<221> FLAG _ CDS
<222> (1004) .. (1027)

<220>
<221> Snap Tag _ CDS
<222> (1046) .. (1591)

<220>
<221> Gène codant pour le récepteur béta 2 adrénergique _ CDS
<222> (1622) .. (2857)

<220>
<221> BGH _ polyA_ signal
<222> (2895) .. (3119)

<220>
<221> Géne de résistance à la néomycine _ CDS
<222> (4003) .. (4797)

<220>
<221> Géne de résistance à l'ampicilline _ CDS
<222> (6299) .. (7159)

<400> 7

gacggatcgg gagatctccc gatcccctat ggtgcactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggag tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagagctct ctggctaact agagaaccca 840
ctgcttactg gcttatcgaa attaatacga ctcactatag ggagacccaa gctggctagc 900
gtttaaactt aagctttgag acatggcctt accagtgacc gccttgctcc tgccgctggc 960
cttgctgctc cacgccgcca ggccggccgc cgctagcggc atcgactaca aggacgacga 1020
tgacaaggcc ggcatcgatg ccatcatgga caaagactgc gaaatgaagc gcaccaccct 1080
ggatagccct ctgggcaagc tggaactgtc tgggtgcgaa cagggcctgc acgagatcaa 1140
gctgctgggc aaaggaacat ctgccgccga cgccgtggaa gtgcctgccc cagccgccgt 1200
gctgggcgga ccagagccac tgatgcaggc caccgcctgg ctcaacgcct actttcacca 1260
gcctgaggcc atcgaggagt tccctgtgcc agccctgcac cacccagtgt tccagcagga 1320
gagctttacc cgccaggtgc tgtggaaact gctgaaagtg gtgaagttcg gagaggtcat 1380
cagctaccag cagctggccg ccctggccgg caatcccgcc gccaccgccg ccgtgaaaac 1440
cgccctgagc ggaaatcccg tgcccattct gatcccctgc caccgggtgg tgtctagctc 1500
tggcgccgtg gggggctacg agggcgggct cgccgtgaaa gagtggctgc tggcccacga 1560
gggccacaga ctgggcaagc ctgggctggg tgatatccag cacagtggcg gccgctcgag 1620
agggcaaccc gggaacggca gcgccttctt gctggcaccc aatagaagcc atgcgccgga 1680
ccacgacgtc acgcagcaaa gggacgaggt gtgggtggtg ggcatgggca tcgtcatgtc 1740
tctcatcgtc ctggccatcg tgtttggcaa tgtgctggtc atcacagcca ttgccaagtt 1800

cgagcgtctg cagacggtca ccaactactt catcacttca ctggcctgtg ctgatctggt 1860
catgggcctg gcagtggtgc cctttggggc cgcccatatt cttatgaaaa tgtggacttt 1920
tggcaacttc tggtgcgagt tttggacttc cattgatgtg ctgtgcgtca cggccagcat 1980
tgagaccctg tgcgtgatcg cagtggatcg ctactttgcc attacttcac ctttcaagta 2040
ccagagcctg ctgaccaaga ataaggcccg ggtgatcatt ctgatggtgt ggattgtgtc 2100
aggccttacc tccttcttgc ccattcagat gcactggtac cgggccaccc accaggaagc 2160
catcaactgc tatgccaatg agacctgctg tgacttcttc acgaaccaag cctatgccat 2220
tgcctcttcc atcgtgtcct tctacgttcc cctggtgatc atggtcttcg tctactccag 2280
ggtctttcag gaggccaaaa ggcagctcca gaagattgac aaatctgagg gccgcttcca 2340
tgtccagaac cttagccagg tggagcagga tgggcggacg gggcatggac tccgcagatc 2400
ttccaagttc tgcttgaagg agcacaaagc cctcaagacg ttaggcatca tcatgggcac 2460
tttcaccctc tgctggctgc ccttcttcat cgttaacatt gtgcatgtga tccaggataa 2520
cctcatccgt aaggaagttt acatcctcct aaattggata ggctatgtca attctggttt 2580
caatcccctt atctactgcc ggagcccaga tttcaggatt gccttccagg agcttctgtg 2640
cctgcgcagg tcttctttga aggcctatgg gaatggctac tccagcaacg gcaacacagg 2700
ggagcagagt ggatatcacg tggaacagga gaaagaaaat aaactgctgt gtgaagacct 2760
cccaggcacg gaagactttg tgggccatca aggtactgtg cctagcgata acattgattc 2820
acaagggagg aattgtagta caaatgactc actgctgtct agagggcccg tttaaacccg 2880
ctgatcagcc tcgactgtgc cttctagttg ccagccatct gttgtttgcc cctcccccgt 2940
gccttccttg accctggaag gtgccactcc cactgtcctt tcctaataaa atgaggaaat 3000
tgcatcgcat tgtctgagta ggtgtcattc tattctgggg ggtggggtgg ggcaggacag 3060
caagggggag gattgggaag acaatagcag gcatgctggg gatgcggtgg gctctatggc 3120
ttctgaggcg gaaagaacca gctggggctc tagggggtat ccccacgcgc cctgtagcgg 3180
cgcattaagc gcggcgggtg tggtggttac gcgcagcgtg accgctacac ttgccagcgc 3240
cctagcgccc gctcctttcg ctttcttccc ttcctttctc gccacgttcg ccggctttcc 3300
ccgtcaagct ctaaatcggg ggctcccttt agggttccga tttagtgctt tacggcacct 3360
cgaccccaaa aaacttgatt agggtgatgg ttcacgtagt gggccatcgc cctgatagac 3420
ggtttttcgc cctttgacgt tggagtccac gttctttaat agtggactct tgttccaaac 3480
tggaacaaca ctcaacccta tctcggtcta ttcttttgat ttataaggga ttttgccgat 3540
ttcggcctat tggttaaaaa atgagctgat ttaacaaaaa tttaacgcga attaattctg 3600
tggaatgtgt gtcagttagg gtgtggaaag tccccaggct ccccagcagg cagaagtatg 3660
caaagcatgc atctcaatta gtcagcaacc aggtgtggaa agtccccagg ctccccagca 3720
ggcagaagta tgcaaagcat gcatctcaat tagtcagcaa ccatagtccc gcccctaact 3780

ccgcccatcc cgcccctaac tccgcccagt tccgcccatt ctccgcccca tggctgacta 3840

attttttta tttatgcaga ggccgaggcc gcctctgcct ctgagctatt ccagaagtag 3900

tgaggaggct tttttggagg cctaggcttt tgcaaaaagc tcccgggagc ttgtatatcc 3960

attttcggat ctgatcaaga gacaggatga ggatcgtttc gcatgattga acaagatgga 4020

ttgcacgcag gttctccggc cgcttgggtg gagaggctat tcggctatga ctgggcacaa 4080

cagacaatcg gctgctctga tgccgccgtg ttccggctgt cagcgcaggg gcgcccggtt 4140

ctttttgtca agaccgacct gtccggtgcc ctgaatgaac tgcaggacga ggcagcgcgg 4200

ctatcgtggc tggccacgac gggcgttcct tgcgcagctg tgctcgacgt tgtcactgaa 4260

gcgggaaggg actggctgct attgggcgaa gtgccggggc aggatctcct gtcatctcac 4320

cttgctcctg ccgagaaagt atccatcatg gctgatgcaa tgcggcggct gcatacgctt 4380

gatccggcta cctgcccatt cgaccaccaa gcgaaacatc gcatcgagcg agcacgtact 4440

cggatggaag ccggtcttgt cgatcaggat gatctggacg aagagcatca ggggctcgcg 4500

ccagccgaac tgttcgccag gctcaaggcg cgcatgcccg acggcgagga tctcgtcgtg 4560

acccatggcg atgcctgctt gccgaatatc atggtggaaa atggccgctt ttctggattc 4620

atcgactgtg gccggctggg tgtggcggac cgctatcagg acatagcgtt ggctacccgt 4680

gatattgctg aagagcttgg cggcgaatgg gctgaccgct tcctcgtgct ttacggtatc 4740

gccgctcccg attcgcagcg catcgccttc tatcgccttc ttgacgagtt cttctgagcg 4800

ggactctggg gttcgaaatg accgaccaag cgacgcccaa cctgccatca cgagatttcg 4860

attccaccgc cgccttctat gaaaggttgg gcttcggaat cgttttccgg gacgccggct 4920

ggatgatcct ccagcgcggg gatctcatgc tggagttctt cgcccacccc aacttgttta 4980

ttgcagctta taatggttac aaataaagca atagcatcac aaatttcaca aataaagcat 5040

ttttttcact gcattctagt tgtggtttgt ccaaactcat caatgtatct tatcatgtct 5100

gtataccgtc gacctctagc tagagcttgg cgtaatcatg gtcatagctg tttcctgtgt 5160

gaaattgtta tccgctcaca attccacaca acatacgagc cggaagcata aagtgtaaag 5220

cctggggtgc ctaatgagtg agctaactca cattaattgc gttgcgctca ctgcccgctt 5280

tccagtcggg aaacctgtcg tgccagctgc attaatgaat cggccaacgc gcggggagag 5340

gcggtttgcg tattgggcgc tcttccgctt cctcgctcac tgactcgctg cgctcggtcg 5400

ttcggctgcg gcgagcggta tcagctcact caaaggcggt aatacggtta tccacagaat 5460

caggggataa cgcaggaaag aacatgtgag caaaaggcca gcaaaaggcc aggaaccgta 5520

aaaaggccgc gttgctggcg tttttccata ggctccgccc ccctgacgag catcacaaaa 5580

atcgacgctc aagtcagagg tggcgaaacc cgacaggact ataaagatac caggcgtttc 5640

cccctggaag ctccctcgtg cgctctcctg ttccgaccct gccgcttacc ggatacctgt 5700

ccgcctttct cccttcggga agcgtggcgc tttctcatag ctcacgctgt aggtatctca 5760
gttcggtgta ggtcgttcgc tccaagctgg gctgtgtgca cgaacccccc gttcagcccg 5820
accgctgcgc cttatccggt aactatcgtc ttgagtccaa cccggtaaga cacgacttat 5880
cgccactggc agcagccact ggtaacagga ttagcagagc gaggtatgta ggcggtgcta 5940
cagagttctt gaagtggtgg cctaactacg gctacactag aagaacagta tttggtatct 6000
gcgctctgct gaagccagtt accttcggaa aaagagttgg tagctcttga tccggcaaac 6060
aaaccaccgc tggtagcggt ttttttgttt gcaagcagca gattacgcgc agaaaaaaag 6120
gatctcaaga agatcctttg atcttttcta cggggtctga cgctcagtgg aacgaaaact 6180
cacgttaagg gattttggtc atgagattat caaaaaggat cttcacctag atccttttaa 6240
attaaaaatg aagttttaaa tcaatctaaa gtatatatga gtaaacttgg tctgacagtt 6300
accaatgctt aatcagtgag gcacctatct cagcgatctg tctatttcgt tcatccatag 6360
ttgcctgact ccccgtcgtg tagataacta cgatacggga gggcttacca tctggcccca 6420
gtgctgcaat gataccgcga gacccacgct caccggctcc agatttatca gcaataaacc 6480
agccagccgg aagggccgag cgcagaagtg gtcctgcaac tttatccgcc tccatccagt 6540
ctattaattg ttgccgggaa gctagagtaa gtagttcgcc agttaatagt ttgcgcaacg 6600
ttgttgccat tgctacaggc atcgtggtgt cacgctcgtc gtttggtatg gcttcattca 6660
gctccggttc ccaacgatca aggcgagtta catgatcccc catgttgtgc aaaaaagcgg 6720
ttagctcctt cggtcctccg atcgttgtca gaagtaagtt ggccgcagtg ttatcactca 6780
tggttatggc agcactgcat aattctctta ctgtcatgcc atccgtaaga tgcttttctg 6840
tgactggtga gtactcaacc aagtcattct gagaatagtg tatgcggcga ccgagttgct 6900
cttgcccggc gtcaatacgg gataataccg cgccacatag cagaacttta aaagtgctca 6960
tcattggaaa acgttcttcg gggcgaaaac tctcaaggat cttaccgctg ttgagatcca 7020
gttcgatgta acccactcgt gcacccaact gatcttcagc atcttttact ttcaccagcg 7080
tttctgggtg agcaaaaaca ggaaggcaaa atgccgcaaa aaagggaata agggcgacac 7140
ggaaatgttg aatactcata ctcttccttt ttcaatatta ttgaagcatt tatcagggtt 7200
attgtctcat gagcggatac atatttgaat gtatttagaa aaataaacaa ataggggttc 7260
cgcgcacatt tccccgaaaa gtgccacctg acgtc 7295

**Description**

L'invention est relative à une méthode de détection de l'internalisation de protéines membranaires par des cellules vivantes et est particulièrement utile pour mettre en évidence des composés capables de provoquer cette internalisation. Plus précisément, la présente invention décrit une méthode de détection de l'internalisation d'une protéine transmembranaire couplée à un complexe métallique fluorescent, basée sur la mesure d'une variation de la luminescence dudit complexe métallique fluorescent lorsqu'il est internalisé.

## ETAT DE LA TECHNIQUE

*INTERNALISATION :*

Lors de l'activation des cellules par des ligands de récepteurs membranaires, ces derniers sont soumis à une cascade de mécanismes qui conduit classiquement à leur internalisation par la cellule. Ce processus permet d'une part, de supprimer ou diminuer le stimulus subi par la cellule par capture du ligand, généralement agoniste, et d'autre part, conduit à une désensibilisation des récepteurs et donc à l'interruption de la cascade de transduction classique du signal. Cette désensibilisation de l'unité de transduction est un mécanisme complexe qui a été relativement bien décrit (voir par exemple Gainetdinov et al., Annu Review Neurosci 2004 v27 p 107-44)

Ce mécanisme d'internalisation est l'un des processus majeurs de régulation des récepteurs couplés aux protéines G (RCPGs) ainsi que des récepteurs à activité tyrosine-kinase.

De manière schématique, l'activation d'un RCPG par un agoniste conduit à la phosphorylation de ce récepteur dans sa partie intracellulaire, ce qui induit le recrutement d'un certain nombre de protéines intracellulaires, en particulier de l'arrestine qui provoque la formation de vésicules d'endocytose recouvertes de clathrine. Ces vésicules sont alors prises en charge par des mécanismes de transport intracellulaire qui conduisent à leur dégradation par fusion avec les lyzosomes, ou bien à leur reconversion par fusion avec la membrane plasmique.

Les RCPGs constituent une cible de choix pour le développement de nouvelles molécules à activité thérapeutique, et plusieurs techniques existent pour mesurer des variations de leur niveau d'activation par des composés candidats : agonistes, antagonistes ou encore agonistes inverses. Certaines d'entre elles ont justement pour objet de détecter l'activation de RCPGs en étudiant des événements liés à leur internalisation.

L'arrestine est une protéine intracellulaire jouant un rôle majeur dans le processus d'internalisation des RCPG et elle est utilisée dans plusieurs approches de détection de l'internalisation ainsi que le montrent les documents ci-après.

La demande WO 01/67106 décrit une méthode visant à identifier des ligands de RCPG, en mesurant la modulation de la liaison entre un RCPG et un mutant constitutivement actif de l'arrestine, dont l'activité est indépendante du niveau de phosphorylation du RCPG, ledit ligand étant marqué par un atome radioactif, une enzyme, une protéine rapporteuse ou un composé fluorescent.

La demande WO 01/59451, décrit une méthode de détection de l'activation d'un RCPG qui utilise la technologie « ICAST » (Intercistronic Complementary Analysis Screening technology ou Technologie de criblage par analyse complémentaire intercistronique) basée sur la complémentation de deux sous-unités d'une enzyme mutante, chaque fragment de l'enzyme étant exprimé sous forme de protéine de fusion avec un RCPG d'une part, et avec par exemple l'arrestine d'autre part. Si une interaction RCPG-arrestine a lieu, l'enzyme va être reconstituée par complémentation et pourra générer un signal détectable. Là encore, il s'agit donc d'analyser l'activation de RCPGs par détection de leur liaison à l'arrestine. Cette technique peut être améliorée en utilisant des mutants de RCPG ou de l'arrestine, comme décrit dans la demande WO 01/58923.

La demande WO 2005/007822 décrit une méthode pour déterminer si un composé module l'interaction entre deux protéines d'intérêt. Cette méthode est également basée sur la détection de la liaison RCPG-arrestine. La méthode présentée repose sur l'utilisation d'un RCPG fusionné, dans sa partie C-terminale, avec un facteur de transcription via une séquence peptidique sensible à une protéase spécifique. L'arrestine est quant à elle fusionnée avec cette protéase. Lors de l'activation du RCPG par un ligand agoniste, l'arrestine va interagir avec le RCPG, ce qui aura pour effet de rapprocher la protéase de la séquence de clivage et de provoquer la libération du facteur de transcription, qui sera alors capable d'activer la transcription d'un gène rapporteur dont l'activité pourra être détectée.

Un des problèmes liés à l'utilisation de la liaison arrestine-RCPG comme marqueur de l'activation d'un RCPG est le caractère instable de cette liaison, qui n'est qu'une étape intermédiaire dans une cascade d'évènements conduisant à la formation de vésicules d'endocytose. La demande WO 04/065963 se propose de résoudre ce problème en utilisant des mutants de l'arrestine qui, contrairement à l'arrestine sauvage ne sont pas capables de se lier avec les partenaires protéiques de cette cascade et par conséquent restent liés de manière relativement stable au récepteur.

Charest et al. (EMBO Rep. 2005 Apr; 6 (4) : 334- 40) ont développé une technique permettant la détection de variation de la structure tridimensionnelle de l'arrestine, qui survient lors de son interaction avec les RCPGs activés ; les auteurs ont construit une protéine de fusion luciférase- arrestine- YFP (« yellow fluorescent protein » ou protéine

fluorescente jaune) et ont mis en évidence une augmentation du signal de BRET (« Bioluminescent Resonance Energy Transfer » ou transfert d'énergie de résonance par bioluminescence) intramoléculaire lors de l'activation de RCPGs. (technique connue sous l'expression « arrestine double- brillance ») .

La société Molecular Devices commercialise la technologie « Transfluor®», décrite notamment dans le brevet EP 1 015 608, qui repose sur l'utilisation de protéine de fusion arrestine-GFP : l'utilisation de cellules exprimant cette protéine de fusion permet de visualiser, avec l'équipement d'acquisition d'images approprié, la translocation d'arrestine-GFP et sa redistribution dans la cellule, en réponse à l'activation d'un RCPG d'intérêt.

D'autres approches de mise en évidence de l'internalisation de récepteurs membranaires qui ne sont pas basées sur la détection de la liaison RCPG-arrestine ou de la translocation intracellulaire d'arrestine ont été décrites notamment dans les documents ci-après.

La demande WO 00/03246 décrit une méthode d'identification de composés capables d'induire l'internalisation de RCPGs, basée sur le marquage luminescent des récepteurs, soit par utilisation d'une protéine de fusion RCPG-protéine luminescente, soit en rajoutant dans le milieu une molécule marquée capable de se lier à ces récepteurs. Cette méthode comprend une étape d'acquisition d'images des cellules qui doivent être analysées pour identifier celles dont les récepteurs ont été internalisés.

La société Amersham Biosciences commercialise des dérivés de cyanine sous le nom commercial CypHer5®; ces fluorophores organiques décrits notamment dans la demande WO 00/75237, ont la propriété de n'être que très faiblement fluorescents à pH neutre, et très fluorescents à pH acide. Ces dérivés de cyanine peuvent être conjugués par exemple avec des anticorps capables de se lier à des RCPGs : lors de l'internalisation de l'anticorps-Cypher5 lié au RCPGs, Cypher5 va passer d'un milieu à pH neutre (milieu extracellulaire) à un milieu à pH acide (endosome) et sa luminescence va augmenter.

*COMPLEXES DE LANTHANIDES ET EXTINCTION DE FLUORESCENCE PAR FRET DYNAMIQUE*

L'utilisation de complexes de lanthanides (également dénommés ci- après « complexes de terres rares ») en tant que composés fluorescents utiles à l'étude de phénomènes biologiques a été développée dans les années 1990 (voir par exemple l'article de Mathis et al, Clin. Chem. 1995 Sep; 41 (9) : 1391- 7) .

Le phénomène de FRET est largement utilisé en biologie, notamment pour étudier des interactions biologiques. Il est basé sur l'utilisation d'un composé donneur fluorescent (par exemple un complexe de lanthanide) et d'un composé accepteur éventuellement fluorescent, chacun couplé à une molécule biologique. Lorsqu'une interaction biologique étudiée provoque le rapprochement des molécules biologiques, et que le composé donneur est excité, un transfert d'énergie a lieu entre le donneur et l'accepteur et va résulter en une variation de la luminescence émise par le milieu réactionnel. Plusieurs sociétés commercialisent des réactifs permettant la mise en oeuvre de cette approche pour étudier des processus biologiques ; par exemple la Demanderesse fournit des composés donneurs et accepteurs ainsi que des trousses pour l'étude de phénomènes biologiques particuliers (détection d'activité enzymatique, dosage de seconds messagers etc...)

Le phénomène de transfert d'énergie dynamique entre deux composés donneur et accepteur diffusant librement en solution dans un milieu à été décrit notamment par D. Thomas et al., (1978), PNAS, 75 :12, 5746-5750. Ce phénomène est souvent désigné par l'acronyme DEFET, qui signifie en langue anglaise « Diffusion Enhanced Fluorescence Energy Transfer ». Contrairement au transfert d'énergie classiquement utilisé pour l'étude de phénomènes biologiques, qui est basé sur l'occurrence d'une interaction biologique qui va rapprocher les composés donneurs et accepteurs, le FRET par transfert dynamique, ou DEFET, repose sur la diffusion libre d'un ou des deux partenaires de FRET dans le milieu réactionnel.

D. Thomas et al. (1978), PNAS, 75 :12, 5746-5750) ont décrit un DEFET entre un complexe de terbium (Tb $(DPA)_3$ et la rhodamine, tous deux en solution, et ont également utilisé le DEFET pour déterminer la distance minimale d'approche entre du $Tb(DPA)_3$ présent dans le milieu aqueux d'une vésicule membranaire et le chromophore éosine de l'éosine-phosphatidylcholine contenue dans la membrane. Ces travaux ont été effectués dans les conditions dites de « limite rapide de diffusion », en particulier en utilisant un fluorophore donneur à durée de vie longue (2,2 msec). Ces conditions permettent notamment d'observer un signal à faible concentration d'accepteur et dans lesquelles l'efficacité du transfert d'énergie ne dépend essentiellement que de la distance minimale d'approche entre le donneur et l'accepteur.

La demande de brevet GB 2 223 096 décrit une méthode de détection d'une molécule (par exemple un anticorps ou un antigène) qui est basée sur le concept même de FRET dynamique dont il est connu qu'il dépend de la constante de diffusion des partenaires de FRET. La méthode décrite consiste en effet à mesurer la variation du FRET dynamique entre un composé donneur et un composé accepteur, cette variation résultant de la modification de la diffusion du donneur ou de l'accepteur dans le milieu suite à son interaction avec la molécule à détecter. Cette méthode nécessite qu'un des composés donneur ou accepteur soit conjugué avec une espèce capable d'interagir avec la molécule à détecter (par exemple le donneur ou l'accepteur est conjugué avec une protéine ou un anticorps).

Zheng et al (J. Am. Chem. Soc. 2005, 127, 16178- 16188) ont mis au point une classe de macrocycles lipophiles

(DTPA- PDA- $C_n$, n=10, 12) capables de complexer des lanthanides et de s'insérer dans les membranes lipidiques de manière non- spécifique, pour des applications de marquage cellulaire et d'utilisation en résonnance magnétique nucléaire (dans le cas de complexes de gadolinium) . Les auteurs ont utilisé la technique de DEFET pour déterminer la localisation cellulaire de ces complexes, en mesurant le signal résultant d'un transfert d'énergie entre des complexes de terbium et la calcéine. Les auteurs ont pu détecter un DEFET entre leurs complexes et la calcéine lorsque celle- ci est ajoutée au milieu extracellulaire (la calcéine est imperméable à la membrane cellulaire) mais pas avec la calcéine intracellulaire (introduite dans les cellules sous forme d'ester neutre de calcéine), et en ont donc conclu que leurs complexes de lanthanides lipidiques s'insèrent sur la face externe de la membrane plasmique. Cette équipe n'a pas étudié les éventuelles variations de signal en cas d'internalisation de leurs complexes de lanthanides.

Le DEFET a été également utilisé par plusieurs équipes pour étudier la structure de protéines incorporant des motifs accepteurs d'énergie et leur interaction avec des composés donneurs d'énergie, pour déterminer les potentiels électrostatiques à la surface de protéines.

*COMPLEXES DE LANTHANIDES ET EXTINCTION DE FLUORESCENCE PAR EFFET REDOX*

L'effet de certains agents réducteurs sur la luminescence des complexes de lanthanides a été décrit dans la littérature :

Kielar et al., par exemple, ont étudié l'extinction dynamique de l'état excité de complexes d'europium et de terbium par différents agents réducteurs (Org. Biomol. Chem., 2007, 5, 2975- 2982), et ont montré que pour les complexes de terres rares étudiés, les composés ayant un potentiel d'oxydation légèrement inférieur à 1 V peuvent avoir un effet extincteur de fluorescence par réduction : c'est le cas par exemple de l'ion iodure (+0, 54V), de l'urate (+0, 59V), l'ascorbate (+0, 30 V) et certains catécholates (+0, 54 V à pH 7) . Ces auteurs ont par ailleurs démontré que l'effet extincteur de fluorescence de ces composés sur les complexes de terbium est plus important que celui observé sur les complexes d'europium, à structure chélatante identique.

La demande WO2008/007089 est relative à une méthode de dosage d'analytes ayant un pouvoir réducteur par des complexes de terbium ou d'europium.

Dhonukshe et al. (Current Biology, Current Science 2007, 17(6), 520-527) décrit le marquage de la protéine transmembranaire PIN par le fluorophore EosFP et suggère qu'un système d'endocytose impliquant la formation d'un manteau de clathrine existe chez les plantes.

Reaves et al. (Journal of Cell Science 1996, 109(4), 749-762) étudie la distribution des protéines membranaires lgp110 et lgp120 dans les cellules, suite à l'addition de wortmanine au milieu extracellulaire.

La demande US 2005/026234 décrit des protéines chimériques indicatrices d'événement de phosphorylation par la protéine kinase C, comprenant deux protéines fluorescentes reliées par un domaine susceptible d'être phosphorylé par la protéine kinase C, et un domaine capable de se lier aux acides aminés phosphorylés.

Root (Proc. Nat. Acad. Sci. USA 1997, 94(11), 5685-5690) présente une étude de l'association de l'actine et de la dystrophine dans les cellules, étude qui utilise un anticorps spécifique de l'actine marqué par un dérivé de la rhodamine en tant qu'accepteur, et un anticorps spécifique de la dystrophine marqué par un complexe de terbium comme donneur.

Maurel et al. (Analytical Biochemistry 2004, 329(2), 253-262) décrit la détection de dimères de protéines membranaires, en l'occurrence de l'hétérodimère constituant le récepteur GABA B, par une technique mettant en oeuvre deux anticorps spécifiques de chaque protéine constituant le dimère et marqués par des composés fluorescent partenaires de FRET.

La demande WO 2008/103393, qui n'est pas entrée en phase européenne, décrit une méthode basée sur un phénomène de FRET entre deux partenaires de FRET dont l'un est lié à une protéine cible, dont on veut étudier la translocation, et l'autre cible un composant constitutif de la membrane plasmique.

Il existe un réel besoin d'une méthode sensible, facile à mettre en oeuvre et adaptée à une application à haut débit, pour détecter l'internalisation de protéines membranaires d'intérêt. Une telle méthode serait particulièrement intéressante pour mettre facilement en évidence des composés capables de provoquer l'internalisation de protéines membranaires, notamment dans les approches visant à détecter de nouveaux médicaments. Par ailleurs, dans la mesure où seulement 10 à 40 % des récepteurs susceptibles d'être internalisés le sont effectivement, la méthode en question doit être suffisamment sensible pour permettre l'observation d'une variation de signal lorsque l'internalisation a lieu.

***DEFINITIONS***

"Composé accepteur de FRET compatible avec un complexe métallique fluorescent": cette expression désigne un composé accepteur de FRET formant un couple de partenaires de FRET avec ledit complexe métallique fluorescent.

"Couple de partenaires de FRET" : cette expression désigne un couple constitué d'un composé fluorescent donneur et d'un composé accepteur; lorsqu'ils sont à proximité l'un de l'autre et lorsqu'ils sont excités à la longueur

d'onde d'excitation du composé fluorescent donneur, ces composés émettent un signal de FRET. Il est connu que pour que deux composés fluorescents soient partenaires de FRET, le spectre d'émission du composé fluorescent donneur doit recouvrir partiellement le spectre d'excitation du composé accepteur. Les couples de partenaire de FRET préférés sont ceux pour lesquels la valeur R0 (distance de Förster, distance à laquelle le transfert d'énergie a une efficacité de 50%) est supérieure ou égale à 30Å.

« Signal de FRET » : désigne tout signal mesurable représentatif d'un FRET entre un composé fluorescent donneur et un composé accepteur. Un signal de FRET peut donc être une variation de l'intensité ou de la durée de vie de luminescence du composé fluorescent donneur ou du composé accepteur lorsque ce dernier est fluorescent.

« Milieu réactionnel » : désigne tout contenant dans lequel peuvent être mises en contact des cellules vivantes avec des réactifs, tel que, par exemple, un puits d'une plaque, un tube à essai, etc.

" EDTA " : acide éthylènediaminetétraacétique ;
" DTPA " : acide diéthylène triamine pentaacétique ;
" TTHA " : acide triéthylènetétramine- N, N, N'N"N"'N"'- hexaacétique ;
" DOTA " : acide 1, 4, 7, 10- tétraazacyclododécane- N, N', N", N"'- tétraacétique ;
" NTA " : acide nitrilotriacétique ;
"HDTA": acide hexaméthylènediamine tétraacétique;
"DTPP": acide diéthylènetriaminepentaphosphonique ;
"EDTP": acide éthylènedinitrilotétrakis (méthylphosphonique) ;
"NTP": acide nitrilotri (méthylènephosphonique) ;
"DOTP": acide 1, 4, 7, 10- tétra- azacyclododécane- N', N", N", 'N"'- tetrakis (méthylènephosphonique) ;
"DO3A": acide 1, 4, 7, 10- tétraazacydododécane trisacétique;
"DOTAGA": 1- (1- carboxy- 3- carboxy- propyl)- 4, 7, 10- (carboxyméthyl)- 1, 4, 7, 10- tétraazacyclododécane;
"DY647": fluorophore à structure pentaméthine commercialisé par la société Dyomics ;
"D2": composé fluorescent accepteur organique commercialisé par la société Cisbio Bioassay ;
"PSB": Solution de tampon phosphate ;
" Lumi4- Tb " : complexe de terbium commercialisé par les sociétés Lumiphore et Cisbio Bioassay ;
"DMEM " : "Dulbecco's Modified Eagle's Medium" Milieu de culture cellulaire, disponible commercialement et utilisé dans de nombreuses applications ;
"NHS" : N- hydroxysuccinimide.

***DESCRIPTION***

La présente invention a pour objet une méthode de détection de l'internalisation de protéines, qui comprend les étapes suivantes :

a) marquage de la protéine d'intérêt par un complexe métallique fluorescent comprenant un lanthanide ou du ruthénium, dont la durée de vie est supérieure à 0,1 ms, de préférence comprise entre 0,5 et 6 ms ;
b) ajout dans le milieu réactionnel d'un composé susceptible de provoquer l'internalisation de la protéine d'intérêt ;
c) ajout dans le milieu réactionnel d'un agent modulateur choisi parmi :

1. un composé accepteur de FRET fluorescent ou non-fluorescent, compatible avec ledit complexe métallique fluorescent, dont la concentration finale dans le milieu réactionnel est supérieure à $10^{-7}$ M, et de préférence comprise entre $10^{-6}$ M et $10^{-3}$ M, et dont le poids moléculaire est inférieur à 50 kD, de préférence compris entre 0,1 et 10 kD ;
2. un agent réducteur dont le potentiel redox est inférieur à +0,1 V et de préférence compris entre 0,25 et 0,75 V ;
3. un agent se liant de manière spécifique par liaison non covalente avec le complexe métallique fluorescent ;
4. un ion métallique qui rentre en compétition avec le lanthanide ou le ruthénium pour former un complexe métallique non-fluorescent ;

d) mesure de la luminescence émise par le milieu réactionnel à la longueur d'onde d'émission du complexe métallique fluorescent et/ou à la longueur d'onde d'émission de l'agent modulateur de l'agent modulateur lorsque celui-ci est un composé accepteur fluorescent ;
e) comparaison du signal mesuré à l'étape d) avec un signal de référence mesuré sur des cellules ayant subi uniquement les étapes a) et c), une différence du signal mesuré à l'étape d) par rapport au signal de référence étant représentative de l'internalisation de la protéine d'intérêt.

Ces étapes essentielles peuvent être mise en oeuvre dans cet ordre (a, b, c, d, e). Ce mode de mise en oeuvre est préféré puisque dans ce cas le composé modulateur n'est pas présent dans le milieu extracellulaire lors de l'internalisation de la protéine d'intérêt, ce qui réduit le risque que du composé modulateur soit présent dans les endosomes.

Lorsque le modulateur est un composé accepteur de FRET, ce qui est le cas dans la mise en oeuvre préférée de l'invention, ou un agent réducteur, ces étapes peuvent également être mise en oeuvre dans l'ordre suivant : a), c), b), d), e).

Par ailleurs, une étape de lavage peut être ajoutée entre l'étape a) et l'étape suivante. Par «étape de lavage », on entend une étape consistant à changer le milieu de culture, une ou plusieurs fois (de préférence 2 ou 3 fois). Cette étape de lavage sert à éliminer du milieu extracellulaire le complexe métallique fluorescent qui ne se serait pas couplé avec la protéine d'intérêt ; cette étape est néanmoins optionnelle puisque que l'invention est basée sur l'observation d'une différence de signal en fonction de l'internalisation. Il est toutefois préférable d'opérer ce lavage pour améliorer la sensibilité de la méthode.

La méthode selon l'invention est basée sur la détection d'un signal émis par un milieu réactionnel contenant des cellules vivantes et les réactifs appropriés, et dont l'intensité varie en fonction de l'internalisation par les cellules d'une protéine membranaire d'intérêt.

Plus précisément, dans la méthode selon l'invention la protéine membranaire d'intérêt est liée, de manière directe ou indirecte et au niveau d'un domaine exposé au milieu extracellulaire, à un complexe métallique fluorescent capable d'émettre un signal luminescent. Le milieu extracellulaire contient un composé (dit « composé modulateur ») dont la présence dans le milieu réactionnel a pour effet de modifier le signal émis par le complexe métallique fluorescent.

L'internalisation de la protéine membranaire d'intérêt modifie l'accessibilité du complexe métallique fluorescent pour le composé modulateur : le complexe métallique fluorescent ne sera plus exposé au milieu extracellulaire, mais à celui de l'intérieur d'une vésicule d'endocytose, ou au cytosol après un certain temps. Les inventeurs ont trouvé que ce changement d'accessibilité du complexe métallique fluorescent pour le composé modulateur se traduit par une variation du signal de luminescence émis par le milieu réactionnel, cette variation étant détectable voire éventuellement quantifiable, et cela malgré le fait que seulement 10 à 40 % des récepteurs transmembranaires susceptibles d'être internalisés le sont effectivement.

Le signal de luminescence émis par le milieu réactionnel correspond à la luminescence émise par le complexe métallique fluorescent ou bien, lorsque le modulateur est composé accepteur de FRET, à celle émise par le modulateur. Dans les deux cas c'est la différence d'accessibilité du complexe métallique fluorescent pour le composé modulateur qui entraîne une différence de la luminescence mesurée dans le milieu réactionnel.

Les réactifs mis en oeuvre dans la méthode selon l'invention ainsi que les variantes de mise en oeuvre de celle-ci vont être maintenant décrites plus en détail.

### *A. <u>Complexe métallique fluorescent</u>*

De manière générale on entend par complexe métallique fluorescent un composé constitué d'un lanthanide ou de ruthénium et d'un agent complexant polydenté, c'est-à-dire comportant au moins 2, et de préférence entre 2 et 9 hétéroatomes donneurs d'électrons, tels que N, O, S, ces atomes formant des liaisons de coordination avec le lanthanide ou le ruthénium. De manière préférée, le complexe métallique fluorescent comprend également un ou plusieurs chromophores constitués de structures aromatiques ; de préférence ces structures aromatiques comprennent 1, 2 ou 3 hétéroatomes choisis parmi N et O, qui jouent le rôle d'atomes de coordination du lanthanide ou du ruthénium.

Un complexe métallique fluorescent approprié aux fins de l'invention doit être stable en termes d'association/dissociation de l'agent complexant et de la terre rare, et de préférence sa constante de formation (Kf) doit être supérieure à $10^{10}$ $M^{-1}$.

De nombreux agents complexants ont été décrits et sont connus de l'homme du métier: on peut citer à titre d'exemples d'agents complexants les composés suivants : EDTA, DTPA, TTHA, DOTA, NTA, HDTA, DTPP, EDTP, HDTP, NTP, DOTP, DO3A, DOTAGA

Des exemples de complexes métalliques fluorescents appropriés aux fins de l'invention sont :

- **Les cryptates de lanthanides** comportant un ou plusieurs motifs pyridine. De tels cryptates de terre rare sont décrits par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de lanthanides sont commercialisés par la société Cisbio Bioassay. On peut citer à titre d'exemples non limitatifs les cryptates d'europium de formules ci-dessous, qui peuvent être couplés au composé à marquer via un groupe réactif, tel que le groupe NHS ou un groupe réactif R.

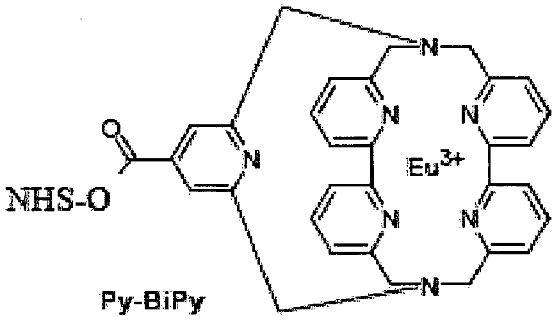

Py-BiPy

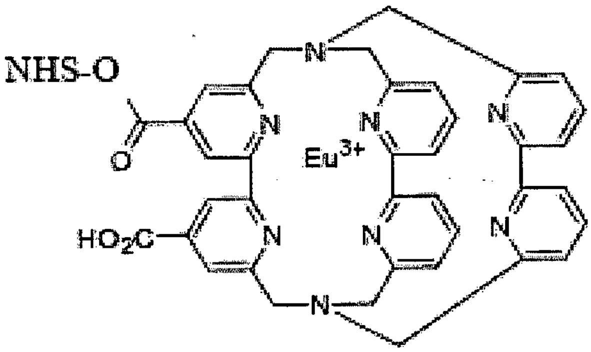

TrisBiPy-Eu

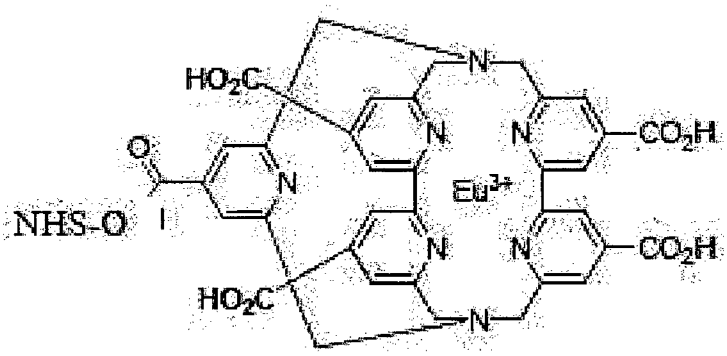

K-Py-BiPy-tetraacide-Eu

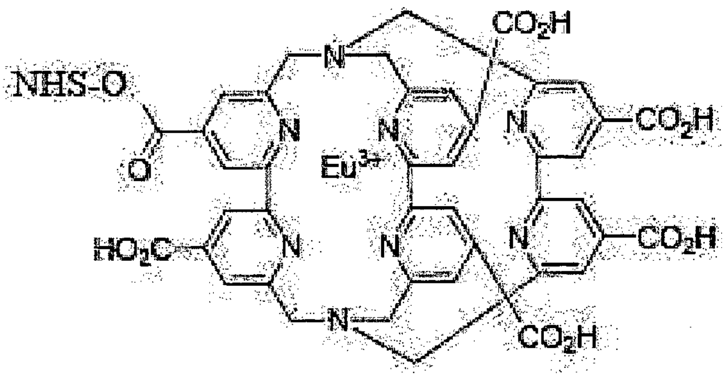

K-TrisBiPy-pentaacide-Eu

- **Les chélates de lanthanides,** décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates composés d'un ligand nonadenté tel que la terpyridine. Des chélates de lanthanides sont commercialisés par la société PerkinElmer.

- **Les complexes de lanthanides constitués d'un agent chélatant** tel que le tetraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes suitable for usage in cellulo", peuvent également être utilisés. On peut aussi utiliser les complexes décrits dans la demande WO2009/10580.

- **Les cryptates de terres rares** décrits dans les brevets EP 1 154 991 et EP 1 154 990 sont également utilisables.

- **Le cryptate de Terbium Lumi4-Tb** de la société Lumiphore, commercialisé par Cisbio bioassays.

- **Le cryptate de terbium Tb(KR)** de formule ci-dessous (qui peut être couplé au composé à marquer via un groupe réactif, ici par exemple un groupe NHS) :

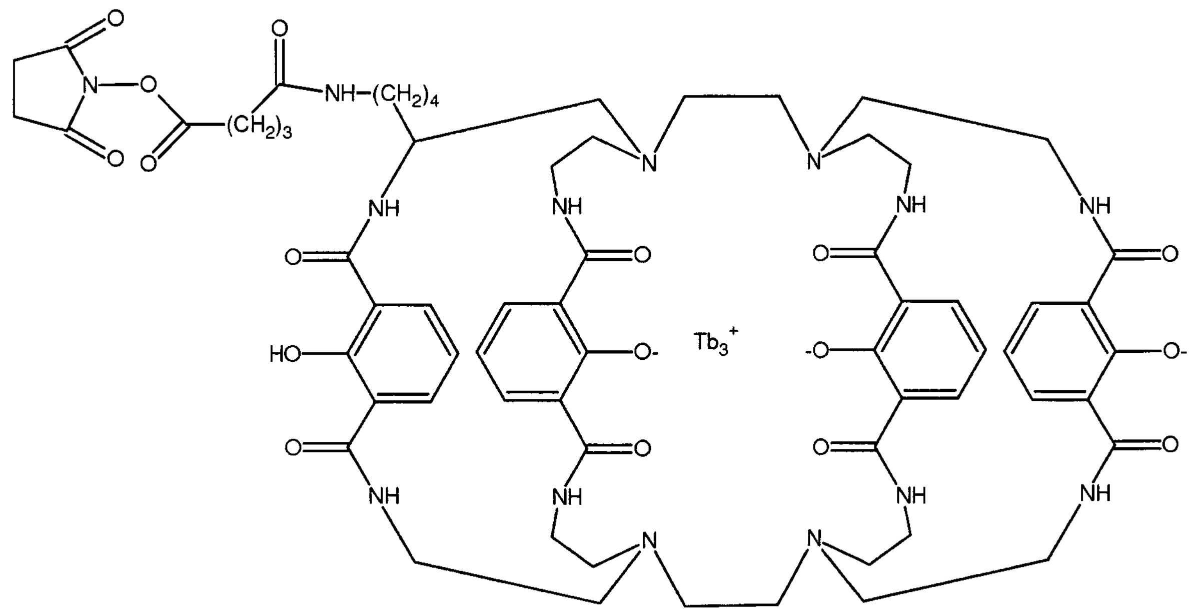

et dont la synthèse est décrite dans la demande internationale WO 2008/063721.

- **Le quantum dye** de la société Research Organics, de formule ci-dessous (qui peut être couplé au composé à marquer via un groupe réactif, ici NCS) :

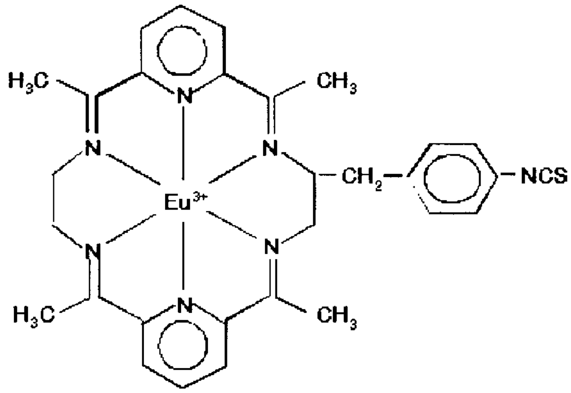

- **Les chélates de ruthénium,** en particulier les complexes constitués d'un ion ruthénium et de plusieurs bipyridines comme le ruthénium (II) tris (2, 2'- bipyridine) .
- **Le chélate de terbium DTPA-cs124 Tb,** commercialisé par la société Invitrogen de formule ci-dessous (qui peut être couplé au composé à marquer via un groupe réactif R) et dont la synthèse est décrite dans le brevet américain US 5,622,821.

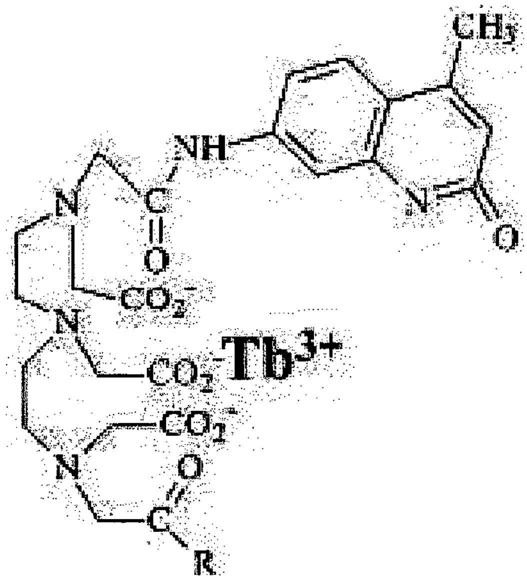

- **Les complexes** de dysprosium (Dy3+), de samarium (Sm3+), de néodymium (Nd3+), d'ytterbium (Yb3+) ou encore d'erbium (Er3+) sont également des complexes de terres rares appropriés aux fins de l'invention, les complexes d'europium (Eu3+) et de terbium (Tb3+) étant particulièrement préférés.

De très nombreux complexes de lanthanides ont été décrits et plusieurs sont actuellement exploités commercialement notamment par les sociétés PerkinElmer, Invitrogen, Molecular Devices et Cisbio Bioassay. La figure 1 montre d'autres exemples de complexes d'europium et de terbium qui peuvent être utilisés aux fins de l'invention.

Bien que l'utilisation d'un complexe métallique fluorescent soit une des caractéristiques essentielles de l'invention, celle-ci n'est pas limitée à un type de complexe métallique fluorescent donné, et l'homme du métier est à même de choisir parmi les complexes de métaux fluorescents, ceux qui sont les plus adaptés à son utilisation particulière et qui ont une durée de vie supérieure à 0,1 ms, de préférence comprise entre 0,5 et 6 ms.

Un complexe métallique fluorescent susceptible d'être utilisé dans la méthode de l'invention est a) un complexe de lanthanide dans lequel le lanthanide est choisi parmi l'europium, le terbium, le néodymium, le samarium et le

dysprosium ; ou b) un complexe de ruthénium. En particulier, on utilise comme complexe métallique fluorescent un chélate ou un cryptate de lanthanide.

Selon l'invention, le complexe métallique fluorescent est conjugué, c'est-à-dire lié de manière covalente, à un agent de couplage qui va permettre le marquage direct ou indirect de la protéine d'intérêt par le complexe métallique fluorescent. Ces agents de couplage sont décrit dans la partie « marquage des protéines d'intérêt » et sont de manière préférée un membre d'un couple ligand/récepteur, tel que par exemple un anticorps anti-tag, la biotine, ou bien un substrat d'une enzyme « suicide », tels qu'un dérivé de la benzylguanine, de la benzylcytosine ou d'un chloroalcane.

La conjugaison d'un complexe métallique fluorescent avec un agent de couplage relève des connaissances générales de l'homme du métier et repose sur l'utilisation de groupes réactionnels. Des complexes de terres rares disponibles commercialement comportent un groupe réactionnel permettant cette conjugaison à un agent de couplage, ou comportent déjà un tel agent de couplage et sont prêts à l'emploi.

***B. Agent modulateur***

B.1 : l'agent modulateur est un accepteur d'énergie

Dans l'une des mises en oeuvre de la méthode selon l'invention, on utilise un agent modulateur capable de modifier le signal émis par le complexe métallique fluorescent par le phénomène de transfert d'énergie de résonance de Förster (FRET) du complexe métallique fluorescent (le donneur d'énergie) vers l'agent modulateur (l'accepteur d'énergie). Ce phénomène bien connu de l'homme du métier est basé sur un transfert d'énergie entre deux composés partenaires de FRET, lorsqu'ils sont à proximité l'un de l'autre, dans le cas présent entre le complexe métallique fluorescent et l'agent modulateur qui diffuse librement dans le milieu réactionnel.

En général, l'utilisation du phénomène de FRET pour étudier des processus biologiques implique que les membres du couple de partenaires de FRET soient chacun conjugués à des composés qui vont interagir entre eux, et ainsi amener les partenaires de FRET à proximité l'un de l'autre, ce qui va générer un signal de FRET. Dans la méthode selon l'invention, l'accepteur d'énergie n'est pas conjugué à un composé quelconque et diffuse librement dans le milieu réactionnel, alors que le complexe métallique fluorescent est couplé de manière directe ou indirecte à la protéine membranaire d'intérêt : le transfert d'énergie entre les deux partenaires de FRET dépend seulement de la diffusion du composé accepteur dans le milieu réactionnel, selon le phénomène de DEFET.

Les inventeurs ont déterminé les conditions nécessaires à l'établissement d'un DEFET entre un complexe métallique fluorescent couplé à une protéine membranaire et un accepteur diffusant dans le milieu extracellulaire, ces conditions permettant également d'observer une variation de signal lorsque la protéine d'intérêt est internalisée.

Selon cette mise en oeuvre, les conditions expérimentales à utiliser pour observer un DEFET dépendant de l'internalisation de la protéine d'intérêt et du complexe métallique fluorescent sont les suivantes :

- le complexe métallique fluorescent et l'agent modulateur sont des partenaires de FRET ;
- la durée de vie du complexe métallique fluorescent doit être supérieure à 0,1 ms, de préférence comprise entre 0,5 et 6 ms.
- la concentration de l'agent modulateur dans le milieu extracellulaire doit être supérieure à $10^{-7}$M, de préférence comprise entre 1 μM et 1 mM. De manière préférée, la concentration de cet agent est comprise entre 1 μM et 100 μM, ce qui permet de travailler dans la limite de diffusion rapide et de mesurer un signal de DEFET indépendant de la concentration en complexe métallique fluorescent.
- l'agent modulateur ne doit pas, de préférence, traverser la membrane cellulaire. De plus, sa constante de diffusion doit être suffisamment élevée. De manière générale, un composé modulateur ayant un poids inférieur à 50 kD, de préférence compris entre 0,1 et 10 kD est approprié aux fins de l'invention.

Dans cette mise en oeuvre de la méthode selon l'invention, l'agent modulateur peut être fluorescent ou pas.

Dans le cas où l'agent modulateur est un composé fluorescent accepteur d'énergie et que le lanthanide est le terbium ou l'europium, cet agent modulateur peut être choisi parmi les composés ou familles de composés suivants : les dérivés de cyanines, DY647, D2, la fluorescéine et ses dérivés, la coumarine, la rhodamine, la carbopyronine, l'oxazine et ses analogues, les AlexaFluors, le crystal violet, les fluorophores de type perylènebisimide, les squaraines, les dérivés du boron- dipyrrométhène, connus sous la dénomination commerciale BODIPY®, le NBD (nitrobenzoxadiazole) et ses dérivés, le DABCYL (acide 4- ((4- (diméthylamino) phényl) azo) benzoïque) .

Selon un mode de réalisation, l'agent modulateur est un composé accepteur fluorescent choisi parmi les composés suivants : la fluorescéine et ses dérivés, les dérivés de cyanine.

Les composés AlexaFluors et les dérivés du boron-dipyrrométhène sont commercialisés par la société Invitrogen; le composé Dy647 est un dérivé de cyanine commercialisé par la société Dyomics ; Les dérivés de cyanine sont également commercialisés notamment par la société Amersham Biosciences ; les autres composés sont commercialisés

par divers fournisseurs de réactifs chimiques, tels que les sociétés Sigma, Aldrich ou Acros.

Des composés non-fluorescents peuvent également être utilisés, sous réserve que leur spectre d'absorption soit compatible avec le spectre d'émission du complexe métallique fluorescent. Les accepteurs non-fluorescents suivants sont disponibles commercialement : les produits QXL de la société Anaspec et en particulier QXL 570, QXL 610, QXL 670 et QXL 680 ; les produits DYQ660 et DYQ661 de la société Dyomics, les produits QSY7, QSY9 et QSY21 de la société Invitrogen.

Le signal détecté dans le milieu réactionnel peut être : la luminescence émise par le complexe métallique fluorescent, ou, dans le cas où le composé accepteur est également fluorescent, la luminescence émise par le composé accepteur, leur intensité ou leur durée de vie. Dans tous les cas, ces signaux dépendent du phénomène de DEFET et vont varier lors de l'internalisation de la protéine transmembranaire d'intérêt, de la manière suivante :

- l'intensité ou la durée de vie de la luminescence du complexe métallique fluorescent mesurée à sa longueur d'onde d'émission va augmenter si la protéine transmembranaire est internalisée ;
- si le modulateur est un composé fluorescent accepteur, l'intensité ou la durée de vie du signal mesuré à la longueur d'onde d'émission de l'accepteur va changer lorsque la protéine est internalisée. Il faut noter que le signal du composé accepteur dépend du DEFET avec le complexe métallique fluorescent, mais également d'éventuels effets d'extinction de luminescence intermoléculaires, se produisant dans le milieu extracellulaire lorsque la concentration en composé accepteur est élevée. Pour cette raison, après internalisation, le signal émis à la longueur d'onde d'émission de l'agent modulateur accepteur peut augmenter ou diminuer.
- si le modulateur est un composé fluorescent accepteur, son signal peut être corrigé en calculant le ratio signal accepteur/signal donneur. Cette méthode de correction ratiométrique du signal de FRET est largement utilisée et décrite dans la littérature.

<u>B2: l'agent modulateur est un agent réducteur pour le complexe métallique fluorescent</u>

Les inventeurs ont découvert que des agents réducteurs capables de diminuer la luminescence des complexes métalliques fluorescents par des réactions d'oxydoréduction au niveau du métal du complexe métallique fluorescent et/ou de la structure complexante, peuvent être avantageusement utilisés dans la méthode selon l'invention.

L'agent réducteur doit avoir un potentiel redox approprié pour réduire le complexe métallique fluorescent. L'homme du métier est à même, selon le complexe métallique fluorescent choisi, de déterminer quels agents réducteurs sont appropriés pour mettre en oeuvre la méthode selon l'invention, mais les agents réducteurs dont le potentiel redox est compris entre + 0,1 et + 1,2 V sont préférés. De manière encore plus préférée, le potentiel redox de l'agent réducteur est compris entre +0,25 et +0,75V.

Ainsi, lorsque l'agent modulateur du signal émis par le complexe métallique fluorescent est un agent réducteur, il peut être choisi par exemple parmi les agents réducteurs suivants : iodures, ascorbates, urates, catécholates, les composés inorganiques tels que $Fe\ (CN)_6^{2+}$. Ces ions sont ajoutés au milieu de mesure sous forme de sels avec un contre- ion, par exemple pour les anions, sous forme de sels de sodium ou de potassium.

Dans cette mise en oeuvre, le signal mesuré va correspondre à la luminescence émise par le complexe métallique fluorescent, modulé par l'agent réducteur présent dans le milieu extracellulaire : lorsque la protéine d'intérêt couplée au complexe métallique fluorescent est internalisée, le complexe métallique fluorescent n'est pas en contact ou à proximité de l'agent modulateur et sa luminescence sera plus importante que dans le cas où l'internalisation n'a pas lieu, de même que sa durée de vie.

Dans cette mise en oeuvre, et pour pouvoir mesurer une différence de signal, entre le cas où il y a internalisation et le cas où l'internalisation n'a pas lieu, les inventeurs ont déterminés que l'agent réducteur doit être présent dans le milieu réactionnel à une concentration supérieure ou égale à la constante de Stern-Volmer du couple complexe métallique fluorescent/agent réducteur. Cette constante est définie comme étant la concentration d'agent réducteur nécessaire pour provoquer une diminution de l'intensité de luminescence de complexe métallique fluorescent de 50%. Des exemples de constante de Stern-Volmer pour des couples complexe métallique fluorescent/agent réducteur sont donnés notamment dans un article de Kielar et al (Org. Biomol. Chem., 2007, 5, 2975-2982).

Ainsi dans un premier aspect de cette mise en oeuvre, c'est la luminescence du complexe métallique fluorescent qui est mesurée.

Dans un deuxième aspect de cette mise en oeuvre, la première étape de la méthode selon l'invention comprend l'ajout dans le milieu de mesure d'un premier complexe métallique fluorescent et d'un second complexe métallique fluorescent destinés à marquer la protéine d'intérêt.

Ce deuxième complexe métallique fluorescent est identique au premier complexe à l'exception du métal qui est différent. Par exemple, le premier complexe métallique fluorescent est un complexe d'europium et le second complexe est un complexe de terbium comportant la même structure chélatante. Dans cette mise en oeuvre, une protéine d'intérêt donnée est donc potentiellement soit liée de manière directe ou indirecte au premier complexe métallique fluorescent,

soit liée de manière directe ou indirecte au deuxième complexe métallique fluorescent (soit n'est marquée par aucun complexe métallique fluorescent).

Dans cette mise en oeuvre il est possible de standardiser le signal mesuré, par exemple en faisant un ratio de la luminescence émise par un des complexes de lanthanides avec l'intensité de la luminescence émise par le second complexe métallique fluorescent. Ce type de mesure permet de comparer les signaux obtenus à partir de milieux réactionnels différents, et est donc particulièrement approprié pour effectuer des études quantitatives.

B3 : l'agent modulateur est un agent se liant de manière spécifique par liaison non covalente sur le complexe métallique fluorescent

L'agent modulateur peut être un agent possédant un domaine permettant une liaison spécifique et non-covalente avec le complexe métallique fluorescent : ces composés peuvent en effet provoquer une diminution de la luminescence du complexe métallique fluorescent. Ces composés peuvent être choisis parmi : des anticorps ou des fragments d'anticorps, des peptides, ou des aptamères, chacun possédant un domaine de liaison au complexe métallique fluorescent.

Parmi ces agents, les modulateurs préférés sont les anticorps ou fragments d'anticorps capables de reconnaître un complexe métallique fluorescent. La demande WO2007/116069(A1) décrit la préparation d'anticorps spécifiques de complexes de terres rares et l'homme du métier dispose par ailleurs de techniques lui permettant de fabriquer ce type de composés.

Pour mettre en oeuvre la méthode selon invention avec ce type de modulateur, il convient de rajouter le composé susceptible de provoquer l'internalisation avant d'ajouter l'agent modulateur, qui dans le cas contraire serait internalisé en même temps que le complexe métallique fluorescent. Autrement dit, dans cette mise en oeuvre, les étapes de la méthode décrite ci-dessus doivent être effectuées dans l'ordre suivant : a), b), c), d), e).

B4 : l'agent modulateur est un ion métallique qui rentre en compétition avec la terre rare pour former un complexe métallique non-fluorescent

Un autre type de modulateur qui convient pour la mise en oeuvre de la méthode selon l'invention est un ion métallique qui va rentrer en compétition avec la terre rare pour former un complexe non-fluorescent avec l'agent complexant. Dans le cas des chélates de terre rare, l'agent modulateur peut ainsi être l'ion Mn2+.

Dans ce cas l'agent modulateur est ajouté au milieu extracellulaire sous forme de sel avec un contre-ion approprié, par exemple sous forme de $MnCl_2$.

Pour mettre en oeuvre la méthode selon l'invention avec ce type de modulateur, il convient de rajouter le composé susceptible de provoquer l'internalisation avant d'ajouter l'agent modulateur. Autrement dit, dans cette mise en oeuvre, les étapes de la méthode décrite ci-dessus doivent être effectuées dans l'ordre suivant : a), b), c), d), e).

## *C. Protéines d'intérêt - Marquage par un complexe métallique fluorescent*

### *C.1. Expression*

L'invention peut être mise en oeuvre avec toutes les protéines membranaires susceptibles d'être internalisées, sous réserve qu'elles puissent être couplées, de manière directe ou indirecte et dans leur partie extracellulaire, avec un complexe métallique fluorescent.

L'invention est particulièrement utile pour étudier l'internalisation des récepteurs couplés aux protéines G (ci-après « RCPG»), et les récepteurs à activité tyrosine-kinase (ci-après « RTK »), dont un des mécanismes de régulation est l'internalisation, comme mentionné précédemment. Il est néanmoins possible de mettre en oeuvre l'invention avec d'autres protéines membranaires dont on veut vérifier si elles sont internalisées ou non.

Les protéines d'intérêt sont exprimées dans les membranes cellulaires de manière naturelle, ou bien sont exprimées en utilisant les techniques classiques de biologie moléculaire, en particulier des vecteurs d'expression introduits dans les cellules de manière stable ou transitoire. Les réactifs destinés à l'introduction d'ADN hétérologue dans des cellules, de manière stable ou transitoire, sont disponibles dans le commerce et les séquences d'ADN codant pour les protéines d'intérêt, en particulier celles codant pour les RCPG et les RTK, sont disponibles dans les bases de données telles que Genbank. Lorsque les protéines d'intérêt sont exprimées par les cellules de manière stable, des phénomènes de cytotoxicité peuvent être observés en raison de la présence d'un trop grand nombre de RCPG ; dans ces cas-là, il peut être intéressant d'utiliser un système d'expression inductible pour limiter l'expression des RCPGs.

Ainsi, la méthode selon l'invention peut comprendre une étape préliminaire de transfection de cellules avec un vecteur d'expression codant pour la protéine d'intérêt, en particulier un RCPG ou un RTK, ce vecteur contenant également la séquence codant pour une protéine de fusion dont la partie N- terminale comprend une enzyme suicide permettant le marquage covalent de la protéine d'intérêt avec le complexe métallique fluorescent, la partie C- terminale comprenant

la protéine d'intérêt.

Dans une mise en oeuvre particulière, un deuxième vecteur d'expression est cotransfecté avec celui codant pour la protéine d'intérêt, ce vecteur d'expression comprenant la séquence codant pour la β-arrestine 1. Bien que cette coexpression ne soit pas indispensable à la mise en oeuvre de l'invention, elle peut permettre d'augmenter la sensibilité de la méthode puisque la β-arrestine 1 peut amplifier de manière non spécifique les phénomènes d'internalisation.

*C.2. Marquage par un complexe métallique fluorescent*

Une mise en oeuvre particulièrement aisée de l'invention consiste à conjuguer le complexe métallique fluorescent à un composé connu (ligand) comme étant capable de se lier à une protéine d'intérêt : le ligand modifié, en se liant à la protéine d'intérêt, permet le marquage indirect de cette protéine.

De nombreuses techniques ont été décrites pour modifier les protéines et l'homme du métier est à même d'utiliser les outils existants pour coupler une protéine à un complexe métallique fluorescent. En particulier les techniques classiques de biologie moléculaire permettent de rajouter, supprimer ou modifier un domaine donné d'une protéine.

La seule caractéristique essentielle à la mise en oeuvre de l'invention est que le complexe métallique fluorescent soit couplé à la protéine de manière à se trouver dans le milieu extracellulaire, pour que le signal de luminescence émis par le milieu réactionnel soit effectivement dépendant de la présence dans le milieu extracellulaire d'un composé modulateur.

Si la protéine d'intérêt est un RCPG ou un RTK, le complexe métallique fluorescent sera donc conjugué à cette protéine via une modification de son domaine N- terminal (extracellulaire), pour permettre son marquage direct ou indirect par le complexe métallique fluorescent selon une des techniques décrite ci- après.

A titre illustratif et non limitatif, on peut citer les techniques suivantes pour coupler une protéine transmembranaire avec un complexe métallique fluorescent :

** Couplage de la protéine d'intérêt avec un complexe métallique fluorescent de manière indirecte (non covalente).*

Le complexe métallique fluorescent peut être couplé avec la protéine d'intérêt par l'intermédiaire d'un couple de partenaires de liaisons dont l'un au moins est de nature protéique. Dans cette approche, la protéine d'intérêt est fusionnée par les techniques classiques de biologie moléculaire avec le partenaire de liaison de nature protéique par les techniques classiques de biologie moléculaire (construction d'un vecteur d'expression comprenant une séquence de nucléotides codant pour la protéine d'intérêt, fusionnée avec celle codant pour le partenaire de liaison protéique, et introduction du vecteur d'expression dans la cellule). Le complexe métallique fluorescent est conjugué de manière covalente à l'autre partenaire de liaison, que l'on appelle ici agent de couplage, qui sera ensuite ajouté au milieu extracellulaire. La reconnaissance des partenaires de liaison permet le marquage indirect de la protéine d'intérêt par le complexe métallique fluorescent.

A titre d'exemple non limitatif de partenaires de liaison particulièrement adaptés à la mise en oeuvre de l'invention on citera :

- le couple constitué de la séquence cystéine- cystéine- X- X- cystéine- cystéine (SEQ ID N°1) dans laquelle X est un acide aminé quelconque et d'un composé bi- arsenic. Ces composés bi- arsenic peuvent être facilement marqués avec une molécule organique du type fluorescéine ou rhodamine (voir B.A.Griffin et al. (1998) Science. 1998, 281, 269- 272 et S.A. Adams et al. (2002) J. Am. Chem. Soc. 2002, 124, 6063- 6076 pour des détails sur la technologie) .
- Le peptide BTX (bungarotoxine), composé d'un peptide de 13 acides aminés qui est reconnu par la bungarotoxine (BTX), peut être couplé à une molécule fluorescente. (voir C. M. McCann et al. (2005), Biotechnique (2005), 38, 945- 952) .
- Le couple streptavidine / Biotine : La séquence de liaison de la Streptavidine (SBP-Tag) est une séquence formée par 38 acides aminés qui présente une haute affinité pour la biotine pouvant être préalablement marquée avec un complexe métallique fluorescent (voir C. M. McCann et al. (2005), Biotechnique (2005), 38, 945-952).
- La séquence de l'enzyme dihydrofolate réductase d'E. coli (eDHFR) qui lie spécifiquement et avec une haute affinité des ligands, tels que la triméthoprime sur lesquels peuvent être greffés le complexe métallique fluorescent selon la technologie dénommée « Ligand link Universal labelling technology » de la Société Active Motif.
- Les couples tags/antitags sont des partenaires de liaison fréquemment utilisés pour marquer des protéines. Le terme « tag » désigne une petite « étiquette » protéique constituée d'un séquences d'acides aminés, généralement mais pas obligatoirement assez courte (moins de 15 acides aminés), qui est fusionnée à la protéine d'intérêt ou bien est naturellement présente dans cette protéine. Le terme antitag désigne un anticorps se liant de manière spécifique audit tag. Dans cette mise en oeuvre, l'anticorps antitag est lié de manière covalente au complexe métallique fluorescent. Lorsque l'anticorps ainsi marqué est ajouté au milieu extracellulaire, il se lie au tag conjugué à la protéine d'intérêt et l'interaction tag/antitag permet le marquage indirect de cette protéine par le complexe

métallique fluorescent. A titre d'exemple non-limitatif de couples tags/antitags, on peut citer les couples suivants dont les membres sont disponibles commercialement : GST/anticorps anti-GST dans lequel GST représente la glutatione S-transférase ou un de ses fragments ; 6HIS/anticorps anti-6HIS dans lequel 6HIS est un peptide constitué de 6 histidines ; Myc/anticorps anti-Myc dans lequel Myc est un peptide constitué des acides aminés 410-419 de la protéine Myc humaine ; FLAG/anticorps anti-FLAG dans lequel FLAG est un peptide ayant les 8 acides aminés DYKDDDDK (SEQ ID N°2) ; HA/anticorps anti HA dans lequel HA est un épitope de l'hémaglutinine d'Influenza, constitué des 9 acides aminés YPYDVPFYA (SEQ ID N°3). Il est clair que la nature exacte du tag n'est pas critique pour la mise en oeuvre de l'invention.

** Couplage de la protéine d'intérêt avec un complexe métallique fluorescent de manière directe (covalente).*

Dans cette approche, le complexe métallique fluorescent va être couplé à la protéine d'intérêt par une liaison covalente ; plusieurs techniques ont été décrites et les réactifs nécessaires à leur mise en oeuvre sont disponibles commercialement. Pour ce couplage, on pourra utiliser l'une quelconque des techniques ci-après :

- Formation d'une liaison covalente au niveau d'un groupe réactif présent sur la protéine d'intérêt, en particulier au niveau d'un des groupes suivants: le groupe amino terminal, le groupe carboxylate terminal, les groupes carboxylate des acides aspartiques et glutamiques, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.

Ces groupes présents sur la protéine d'intérêt peuvent former une liaison covalente avec un groupe réactif porté par le complexe métallique fluorescent.

Les groupes réactifs appropriés sont connus de l'homme du métier : un complexe métallique fluorescent fonctionnalisé par un groupe maléimide sera par exemple capable de se lier de manière covalente avec les groupes thiols portés par les cystéines de la protéine. De même, un complexe métallique fluorescent portant un ester de N-hydroxysuccinimide sera capable de se fixer de manière covalente à une amine de la protéine d'intérêt.

• Utilisation d'une enzyme suicide :

Par enzymes suicides on entend des protéines qui ont une activité enzymatique modifiée par des mutations spécifiques qui leur confèrent la capacité de lier rapidement et de façon covalente un substrat. Ces enzymes sont dites suicides car chacune ne peut lier qu'une seule molécule fluorescente, l'activité de l'enzyme étant bloquée par la fixation du substrat. Ces enzymes constituent par conséquent un outil de choix pour marquer spécifiquement des protéines d'intérêt avec un rapport d'une molécule fluorescente pour une protéine. Dans cette approche, une enzyme suicide est fusionnée avec la protéine membranaire, de préférence dans sa partie N-terminale par les techniques classiques de biologie moléculaire et le substrat de l'enzyme, lié de manière covalente à un donneur/accepteur, est introduit dans le milieu extracellulaire. La réaction enzymatique a pour conséquence la liaison covalente du substrat marqué à l'enzyme, et donc le marquage de la protéine membranaire par le donneur ou l'accepteur.

On peut citer à titre d'exemple non limitatif les enzymes suivantes :

- les mutants de l'O6-alkylguanine DNA alkyltransférase (AGT). Les enzymes SNAP-tag (Juillerat et al, Chemistry & biology, Vol.10, 313-317 Avril 2003) et CLIP-tag (Gautier et al, Chemistry et Biology, 15, 128-136, février 2008) commercialisées par la société NEB sont des mutants de l'AGT humaine dont les substrats sont respectivement, l'O6-benzylguanine (ci-après abrégée BG) et l'O2-benzylcytosine (ci-après abrégée BC). L'enzyme N-AGT (Gronemeyer et al (Protein engineering, design & selection, vol. 19, no 7, pp 309-316, 2006) est un autre mutant de cette enzyme dont la réactivité avec l'O6-benzylguanine est meilleure que celle de l'enzyme SNAP-tag.
- le mutant d'une déhalogénase (HaloTag commercialisé par Promega) qui génère également une réaction enzymatique du type suicide (voir WO 04/072232 A2), dont certains des substrats sont des composés de la famille des chloroalcanes, en particulier les chloroalcanes comportant le motif- $NH- CH_2CH_2- O- CH_2CH_2- O- (CH_2)_6- Cl$. Dans ce cas, le complexe métallique fluorescent sera conjugué à ce type de motif ;
- La protéine ACP (« Acyl Carrier Protein »), protéine transporteur d'acyles, sur laquelle est transféré, en présence de phosphopanthéthéine transférase, le résidu 4'-phosphopantéthéine du coenzyme A sur une sérine de l'ACP (N.George et al, Journal of the American Chemical society, 126, (2004) p 8896-8897). Lorsque cette approche est utilisée pour marquer la protéine d'intérêt par le complexe métallique fluorescent, il est nécessaire de rajouter au milieu réactionnel de la phosphopantéthéine transférase. La société NEB commercialise un fragment de l'ACP sous le nom commercial « ACP-Tag » pour le marquage de protéines.

Lorsque cette approche est utilisée pour marquer la protéine d'intérêt, les cellules sont transfectées avec un plasmide d'expression comportant l'ADN codant pour une protéine de fusion comprenant l'enzyme suicide et la protéine d'intérêt. Ce plasmide peut également comprendre, en amont de l'ADN codant pour ces protéines, l'ADN codant pour une étiquette telle que par exemple l'épitope FLAG, l'épitope myc, ou encore celui de l'hémaglutinine de l'influenza (HA).

Pour s'assurer que la protéine de fusion sera exprimée dans la membrane cellulaire, il peut être utile d'inclure dans le plasmide d'expression, en amont de la séquence codant pour la protéine d'intérêt et de l'enzyme suicide, celle codant pour un peptide d'adressage membranaire, telle que le peptide signal T8 ou le peptide signal du récepteur mGluR5, dont l'utilisation à cet effet est connue de l'homme du métier. Enfin, il peut également être souhaitable de s'assurer que la séquence codant pour la protéine d'intérêt ne comporte pas de séquence d'adressage membranaire native qui pourrait faire l'objet d'un clivage post-traductionnel de la liaison entre le protéine d'intérêt et l'enzyme suicide : si c'est le cas, il est préférable de ne pas introduire ce domaine dans le plasmide d'expression.

Pour que la réaction enzymatique ait lieu avec le substrat de l'enzyme présent dans le milieu extracellulaire (tel qu'un conjugué BG-complexe métallique fluorescent), il est nécessaire que l'enzyme suicide soit exposée au milieu extracellulaire : lorsque la partie N-terminale de la protéine d'intérêt naturelle est exposée au milieu extracellulaire, ce qui est le cas pour les RCPG et les RTK, la protéine de fusion sera construite de telle sorte que l'enzyme suicide soit exprimée dans la partie N-terminale de la protéine de fusion, mais toujours en aval du peptide d'adressage membranaire s'il est présent.

Finalement, lorsque une enzyme suicide est utilisée pour marquer la protéine d'intérêt avec le complexe métallique fluorescent, et que la protéine d'intérêt est un RCPG ou un RTK, l'invention comprend une étape préliminaire de transfection des cellules par un vecteur d'expression comprenant la séquence d'ADN codant pour une protéine de fusion dont la partie N- terminale comprend une enzyme suicide et la partie C- terminale comprend la protéine d'intérêt.

L'introduction dans le milieu extracellulaire du substrat de l'enzyme conjugué à un complexe métallique fluorescent aura pour conséquence le marquage de la protéine d'intérêt par ce complexe métallique fluorescent.

Dans cette mise en oeuvre, les vecteurs d'expression codant pour une protéine de fusion choisie parmi les protéines de fusion suivantes peuvent être utilisés :

enzyme suicide - protéine d'intérêt, ou
étiquette - enzyme suicide - protéine d'intérêt, ou
peptide d'adressage membranaire - étiquette - enzyme suicide - protéine d'intérêt.

Selon une mise en oeuvre de la méthode de l'invention, le complexe métallique fluorescent est un cryptate d'europium ou de terbium lié de manière covalente à la benzylguanine ou la benzylcytosine ou un de leurs dérivés, et la protéine d'intérêt est exprimée sous forme de protéine de fusion avec un mutant de l'alkylguanine-DNA alkyltransférase.

Selon une autre mise en oeuvre de la méthode de l'invention, le complexe métallique fluorescent est un cryptate de terbium lié de manière covalente à la benzylguanine ou la benzylcytosine, la protéine d'intérêt est exprimée sous forme de protéine de fusion avec un mutant de l'alkylguanine-DNA alkyltransférase, et le composé modulateur est la fluorescéine ou un de ses dérivés.

## EXEMPLES

Les exemples suivants illustrent des mises en oeuvre préférées de l'invention, à savoir celles dans lesquelles le complexe métallique fluorescent est un cryptate d'europium ou de terbium lié de manière covalente à la benzylguanine ou la benzylcytosine ou un de leurs dérivés, dans lesquelles la protéine d'intérêt est exprimée sous forme de protéine de fusion avec un mutant de l'alkylguanine-DNA alkyltransférase ; en particulier celles dans lesquelles le complexe métallique fluorescent est un cryptate d'europium ou de terbium lié de manière covalente à la benzylguanine ou la benzylcytosine, la protéine d'intérêt est exprimée sous forme de protéine de fusion avec un mutant de l'alkylguanine-DNA alkyltransférase, et l'agent modulateur est un dérivé de cyanine, en particulier la Cy5 ou la Cy5-COOH, ou la fluorescéine ou un de ses dérivés. Lorsqu'un cryptate d'europium est utilisé, il est préférable d'utiliser les dérivés de cyanine plutôt que la fluorescéine.

### Exemple 1 : Détection de l'internalisation du récepteur de la vasopressine V2-R

On se propose dans cet exemple de détecter l'internalisation d'une protéine de fusion comprenant le récepteur V2 de la vasopressine, l'enzyme Snaptag et le tag HA, par la méthode selon l'invention.

Plus précisément, on utilise ici un conjugué cryptate de terbium (LUMI4- TB)- benzylguanine (BG) comme composé donneur, la cyanine Cy5- COOH comme accepteur, et l'on observe les variations de signal résultant d'une variation du DEFET consécutif à l'internalisation du récepteur.

**Réactifs et matériels utilisés**

Milieu OptiMEM (Invitrogen (51985- 026) )
Tampon phosphate PBS
DMEM SRP (Milieu de culture DMEM sans rouge de phénol,Invitrogen)
DMEM+ : DMEM auquel ont été rajoutés : L- alanyl/L- glutamine 1mM, NaPyruvate 1mM, sérum de veau foetal (Invitrogen SKU#10091- 148) 10%, Pénicilline Streptomycine 1% (Invitrogen SKU#15070- 063), HEPES 2 mM, Acides Aminés Non Essentiels 1%)
DMEM SRP+ (Milieu DMEM+, sans rouge de phénol)
Krebs-Tris + glucose 0,5 g/l
BG-LUMI4-TB (conjugué cryptate de terbium benzylguanine, commercialisé par Cisbio Bioassay)
BG : $O^6$-benzylguanine (Sigma B2292)
Vasopressine (Bachem)
Plasmide HA-ST-V2 : plasmide comportant la séquence codant pour une protéine de fusion comprenant un peptide signal d'adressage membranaire T8, l'épitope HA, l'enzyme SNAPTAG et le récepteur V2.
Cyanine Cy5-COOH : Eras Laboratories

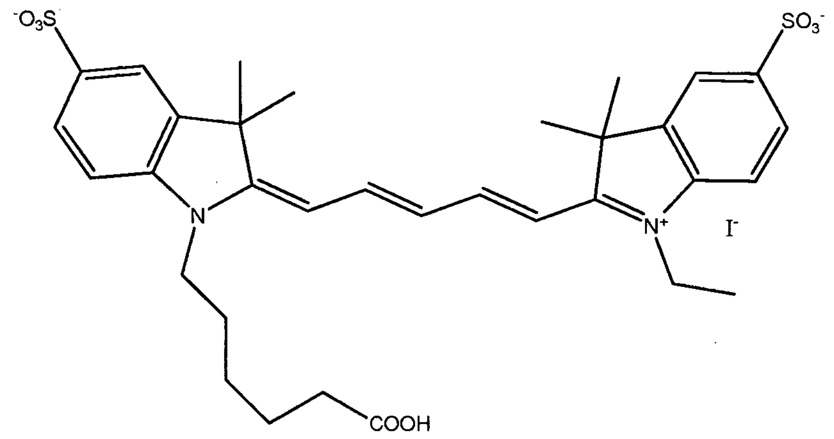

Cy5-COOH

**Traitement des plaques**

50 µl de solution de poly- L- Ornithine (solution 0.01%, poids moléculaire 30, 000- 70, 000 (SIGMA P4957) sont distribués dans chaque puits d'une plaque de 96 puits (Cellstar, noires à fond noir) afin de favoriser l'adhérence des cellules au fond du puits, et les plaques sont mises à incuber pendant 30 min à 37° C, avant d'être rincées 1 fois avec 100 µl de DMEM+.

**Transfection**

Le mélange de transfection suivant ou un mélange contrôle négatif de composition ci-après sont ensuite distribués dans les puits :

| **Mélange de transfection (par puits) :** | **Mélange de contrôle négatif (par puits):** |
|---|---|
| 0,32·µg plasmide HA-ST-V2 (CIS BIO) | 0,32 µg Plasmide PRK6 (CIS BIO) |
| 0,8 µl Lipofectamine 2000 | 0,8 µl Lipofectamine 2000 (Invitrogen) |
| 50 µl Milieu Biologique (DMEM+) | 50 µl milieu Biologique (DMEM+) |

Après 30 minutes d'incubation, 100 µl de cellules COS7 (CIS BIO), soit 80000 cellules sont rajoutées dans

chaque puits, puis incubées pendant 24 heures à 37°C en présence de 5% de $CO_2$.

**<u>Courbe de dose réponse</u>**

Les puits sont rincés une fois avec du milieu DMEM SRP+, avant l'ajout d'une solution de 156 nM de BG-LUMI4-TB ou de 156 nM de BG (contrôle négatif) dans du milieu DMEM SRP+ (100µl), et la plaque est ensuite incubée pendant 1h30.

Chaque puits est ensuite lavé 3 fois avec 100 µl du tampon de Krebs-Tris + glucose (0.5 g/l), avant l'ajout de tampon Krebs-Tris, puis de vasopressine (agoniste du récepteur V2) diluée avec le tampon Krebs-Tris (4°C, 10µl, concentration variable, voir plan de plaque).

Après 30 minutes d'incubation, 264 µM de Cyanine5-COOH en solution dans le tampon Krebs-Tris (10µl) sont ajoutés dans les lignes B, D, E-H (voir plan) pour obtenir une concentration finale d'accepteur de 24 µM.

La plaque est agitée pendant 15 minutes avant d'être lue sur un lecteur Envision (PerkinElmer), équipé d'un laser azote 337 nm et avec des filtres d'émission 545 nm et 665 nm. La lecture est effectuée dans les deux canaux avec un délai de 400 µs et une fenêtre de lecture entre 400 et 2900 µs.

Le plan de la plaque à 96 puits est représenté ci-dessous avec les quantités de réactifs ajoutés dans chaque puits :

<table>
<tr><th></th><th>1-4</th><th colspan="2">5-8</th><th>9-12</th></tr>
<tr><td>A</td><td>PRK6<br>BG (110 µl, 156 nM)</td><td colspan="2">PRK6<br>BG-LUMI4-TB (110 µl, 156 nM)</td><td>PRK6<br>BG-LUMI4-TB (100 µl, 156 nM)<br>Vasopressine (10µl 10µM)</td></tr>
<tr><td>B</td><td>PRK6<br>BG (100 µl, 156 nM)<br>Cy5 (10µl, 264 µM)</td><td colspan="2">PRK6<br>BG-LUMI4-TB (100 µl, 156 nM)<br>Cy5 (10µl, 264 µM)</td><td>PRK6<br>BG-LUMI4-TB (90 µl, 156 nM)<br>Vasopressine (10µl 10µM)<br>Cy5 (10µl, 264 µM)</td></tr>
<tr><td>C</td><td>HA-ST-$V_2$<br>BG (110 µl, 156 nM)</td><td colspan="3">HA-ST-$V_2$<br>BG-LUMI4-TB (110 µl, 156 nM)</td></tr>
<tr><td>D</td><td>HA-ST-$V_2$<br>BG (100 µl, 156 nM)<br>Cy5 (10µl, 264 µM)</td><td colspan="3">HA-ST-$V_2$<br>BG-LUMI4-TB (100 µl, 156 nM)<br>Cy5 (10µl, 264 µM)</td></tr>
<tr><td>E</td><td colspan="2">HA-ST-$V_2$<br>BG (90 µl, 156 nM)<br>Cy5 (10µl, 264 µM)<br>Vasopressine (10µl, 10pM)</td><td colspan="2">HA-ST-$V_2$<br>BG (90 µl, 156 nM)<br>Cy5 (10µl, 264 µM)<br>Vasopressine (10µl, 100pM)</td></tr>
<tr><td>F</td><td colspan="2">HA-ST-$V_2$<br>BG (90 µl, 156 nM)<br>Cy5 (10µl, 264 µM)<br>Vasopressine (10µl, 1nM)</td><td colspan="2">HA-ST-$V_2$<br>BG (90 µl, 156 nM)<br>Cy5 (10µl, 264 µM)<br>Vasopressine (10µl, 10nM)</td></tr>
<tr><td>G</td><td colspan="2">HA-ST-$V_2$<br>BG (90 µl, 156 nM)<br>Cy5 (10µl, 264 µM)<br>Vasopressine (10µl, 100nM)</td><td colspan="2">HA-ST-$V_2$<br>BG (90 µl, 156 nM)<br>Cy5 (10µl, 264 µM)<br>Vasopressine (10µl, 1µM)</td></tr>
<tr><td>H</td><td colspan="2">HA-ST-$V_2$<br>BG (90 µl, 156 nM)<br>Cy5 (10µl, 264 µM)<br>Vasopressine (10µl, 10µM)</td><td colspan="2">HA-ST-$V_2$<br>BG (90 µl, 156 nM)<br>Cy5 (10µl, 264 µM)<br>Vasopressine (10µl, 100µM)</td></tr>
</table>

Ligne A : contrôle négatif avec le plasmide PRK6, donc pas de marquage Snaptag (ST), signal observé en l'absence d'accepteur Cy5, en l'absence de donneur LUMI4-TB (A1-A4), en présence de donneur LUMI4-TB sans vasopressine (A5-A8) ou avec vasopressine (A9-A12)

Ligne B : contrôle négatif avec plasmide PRK6, donc pas de marquage Snaptag (ST), signal observé en présence d'accepteur Cy5, en l'absence de donneur LUMI4- TB (B1- B4), en présence de donneur LUMI4- TB sans vasopressine (B5- B8) ou avec vasopressine (B9- B12)

*Cette ligne permet d'estimer le bruit de fond en l'absence de marquage du récepteur et permet de corriger les signaux mesurés*

Ligne C : contrôle négatif avec le plasmide HA-ST-$V_2$, donc en présence de Snaptag, mais sans vasopressine ni accepteur Cy5, signal observé en présence de BG non fluorescent (C1-C4), ou fluorescent avec le donneur LUMI4-TB (C5-C12).

Ligne D : contrôle négatif avec plasmide HA- ST- $V_2$, donc en présence de Snaptag, mais sans vasopressine, et en présence d'accepteur Cy5 ; signal observé en présence de BG non fluorescent (D1- D4), ou fluorescent avec le donneur LUMI4- TB (D5- D12) .

*Cette ligne permet d'obtenir le signal de référence en l'absence d'internalisation du récepteur V2 marqué par BG-LUMI4- TB.*

Les lignes E à H correspondent au signal observé en présence de différentes concentrations de vasopressine.

**Résultats & Discussion**

La figure 2 représente l'évolution de la luminescence du donneur, mesurée à 545 nm en fonction de la concentration en vasopressine et donc en fonction de l'internalisation du récepteur.

ΔAS% correspond au pourcentage d'augmentation du signal du donneur au cours de l'internalisation, par rapport au signal de référence mesuré sans internalisation (sans vasopressine).

Plus précisément, pour une concentration donnée de vasopressine, par exemple 10 pM (puits E1-E6), ΔAS% est calculé de la façon suivante :

$$\Delta AS\% = \frac{([E1\text{-}E6]_{545} - [D5\text{-}D12]_{545})}{([D5\text{-}D12]_{545} - [D1\text{-}D4]_{545}) - ([B5\text{-}B8]_{545} - [B1\text{-}B4]_{545})}$$

Dans laquelle « $[E1\text{-}E6]_{545}$», « $[D5\text{-}D12]_{545}$ » etc ... correspondent à la moyenne des signaux mesurés dans les puits E1 à E6, D5 à D12 etc ... à la longueur d'onde du donneur BG- LUMI4- TB (545nm)

Le numérateur correspond à la différence entre les signaux mesurés en présence et en l'absence de vasopressine (et donc d'internalisation) et le dénominateur correspond au signal de référence en l'absence d'internalisation ($[D5\text{-}D12]_{545}$) et corrigé par le bruit de fond ($[D1\text{-}D4]_{545}$, mesuré en l'absence de donneur BG- LUMI4- TB) ainsi que par le signal non spécifique ($[B5\text{-}B8]_{545}$- $[B1\text{-}B4]_{545}$ en l'absence de marquage Snaptag)

La figure 2 montre une variation (dans ce cas une augmentation) de la luminescence du composé donneur BG-LUMI4-TB à 545 en présence de concentrations croissantes de vasopressine et valide donc l'utilisation de la méthode selon l'invention pour mettre en évidence l'internalisation de récepteurs membranaires.

Des résultats similaires sont obtenus si l'on s'intéresse au signal émis par l'accepteur à 665 nm (Cy5), corrigé par le signal du donneur à 545 (cette correction ratiométrique permet de s'affranchir de la variabilité du signal d'un puits à l'autre).

La figure 3 représente l'évolution de ΔASR% en fonction de la concentration en vasopressine et donc de l'internalisation du récepteur V2.ΔASR% correspond au pourcentage du signal de référence sans internalisation (mesuré en l'absence de vasopressine)

Pour une concentration donnée de vasopressine, par exemple 10 pM (puits E1-E6), ΔASR% est calculé de la façon suivante :

$$\frac{[E1\text{-}E6]_{665} - [D1\text{-}D4]_{665} - ([B5\text{-}B8]_{665} - [B1\text{-}B4]_{665})}{[E1\text{-}E6]_{545} - [D1\text{-}D4]_{545} - ([B5\text{-}B8]_{545} - [B1\text{-}B4]_{545})}$$

$$\Delta ASR\% = 1- \frac{[D5\text{-}D12]_{665} - [D1\text{-}D4]_{665} - ([B5\text{-}B8]_{665} - [B1\text{-}B4]_{665})}{[D5\text{-}D12]_{545} - [D1\text{-}D4]_{545} - ([B5\text{-}B8]_{545} - [B1\text{-}B4]_{545})}$$

Le signal mesuré en présence de vasopressine ([E1- E6] $_{665}$/ [E1- E6] $_{545}$) et le signal de référence ([D1- D12] $_{665}$/ [D1- D12] $_{545}$) sont chacun corrigés par le bruit de fond ([D1- D4], mesuré en l'absence de donneur BG- LUMI4-TB) et par le signal non spécifique ([B5- B8]- [B1- B4] ) mesuré en l'absence de marquage Snaptag.

La figure 3 montre une variation (une diminution du signal émis par le composé accepteur) lorsque des concentrations croissantes de vasopressine sont présentes dans le milieu, et valide donc l'utilisation de la méthode selon l'invention pour mettre en évidence l'internalisation de récepteurs membranaires.

__Exemple__ **2** : **Détection de l'internalisation du récepteur de la vasopressine V2-R**

Cet exemple illustre une autre mise en oeuvre de la méthode selon l'invention pour détecter l'internalisation du récepteur de la vasopressine V2. En particulier, la fluorescéine a été utilisée comme accepteur et le plasmide d'expression codant pour le récepteur V2 a été cotransfecté avec un plasmide codant pour la β-arrestine 1. Cette cotransfection a permis de favoriser l'internalisation des récepteurs et donc d'augmenter la sensibilité de la méthode.

**Réactifs et matériels utilisés**

Milieu OptiMEM (Invitrogen (51985- 026) )
Krebs-glucose : Tampon Krebs + glucose 0.5g/l
BG-LUMI4-TB (conjugué cryptate de terbium benzylguanine, commercialisé par Cisbio Bioassay)
BG : $O^6$-benzylguanine (Sigma B2292)
Vasopressine (Bachem)
Plasmide FLAG-ST-V2 : plasmide comportant la séquence codant pour une protéine de fusion comprenant un peptide signal d'adressage membranaire T8, l'épitope FLAG, l'enzyme SNAPTAG et le récepteur V2. La séquence de ce plasmide est la SEQ ID N°4
Plasmide Flag-Arrestine β1 : plasmide comportant la séquence codant pour une protéine de fusion comprenant un l'épitope FLAG et la β-arrestine 1 humaine. La séquence de ce plasmide est la SEQ ID N° 5
Fluorescéine (Sigma)

**Traitement des plaques**

50 μl de solution de poly- L- Ornithine (solution 0.01%, poids moléculaire 30, 000- 70, 000 (SIGMA P4957) ont été distribués dans chaque puits d'une plaque de 96 puits (Cellstar, noires à fond noir) afin de favoriser l'adhérence des cellules au fond du puits, et les plaques ont été mises à incuber pendant 30 min à 37° C.

**Transfection**

Le mélange de transfection suivant a ensuite été distribué dans chaque puits :

- 0.16 μg de plasmide FLAG-ST-V2 + 0.16 μg de plasmide Flag-β1 Arrestine
- 0,8 μl Lipofectamine 2000
- 50 μl de milieu OptiMEM

Après 30 minutes d'incubation, 100 μl d'une suspension contenant 100 000 cellules COS7 ont été ajoutées dans chaque puits, puis incubées pendant 24 heures à 37°C en présence de 5% de $CO_2$.

**Courbe de dose réponse**

Le milieu de transfection a été aspiré et une solution de 100 nM de BG-LUMI4-TB dans du milieu Krebs-glucose a été ajoutée à chaque puits. La plaque a ensuite été incubée pendant 1h à 37° en présence de $CO_2$.

Chaque puits a ensuite été lavé 4 fois avec 100 μl de Krebs-glucose avant l'ajout de 90 μl de tampon Krebs-

glucose ou d'une solution de vasopressine.

Après 30 minutes d'incubation à +37°C et 5% de $CO_2$, 10 µl de fluorescéine en solution à 240 µM dans du tampon Krebs glucose ont été ajoutés dans les puits pour obtenir une concentration finale d'accepteur de 24 µM.

La plaque a ensuite été agitée légèrement puis lue sur un lecteur Rubystar (BMG), équipé d'un laser azote 337 nm et avec des filtres d'émission à 620 et 520 nm. La lecture a été effectuée pour le canal accepteur (520 nm) avec un délai de 60 µs et une fenêtre de lecture de 60 à 400 µs et pour le canal donneur (620 nm) avec un délai de 400 µs pour une fenêtre de 400 à 1500 µs.

**<u>Résultats & Discussion</u>**

La figure 4 représente l'évolution du ratio du signal du donneur (mesuré à 620 nm) sur celui de l'accepteur (mesuré à 520 nm), en fonction de la concentration en vasopressine (AVP). Ce ratio permet de s'affranchir des variations inter-puits. Par ailleurs, il a été multiplié par 10 000 pour en faciliter la représentation graphique.

La figure 4 montre très clairement qu'une concentration croissante de vasopressine provoque une augmentation du ratio, correspondant à une augmentation de la luminescence du composé donneur.

L'ajout de vasopressine provoque en effet l'internalisation des récepteurs V2 auxquels sont liés les conjugués donneurs BG- LUMI4- Tb, et par conséquent une augmentation de leur luminescence puisqu'ils ne sont plus impliqués dans des phénomènes de DEFET avec la fluorescéine en solution dans le milieu extracellulaire.

Cet exemple montre donc encore une fois l'excellente corrélation entre l'augmentation du signal du composé donneur correspondant à une baisse de DEFET et l'internalisation du récepteur V2 en présence de son agoniste, la vasopressine. Cet exemple montre également comment la méthode selon l'invention peut être utilisée pour mettre en évidence des composés agonistes d'un récepteur d'intérêt.

**<u>Exemple 3: Effet de l'antagoniste EDA9 du récepteur V2 sur son internalisation induite par la vasopressine.</u>**

Cet exemple illustre l'utilisation de la méthode selon l'invention pour mettre en évidence un composé antagoniste du récepteur V2 de la vasopressine. On utilise pour cela un composé antagoniste connu de ce récepteur, l'EDA9 (PolyPeptide- group) .

Les réactifs et matériels utilisés sont les mêmes que ceux de l'exemple 2. Il en est de même du traitement des plaques et des réactifs et protocoles de transfection.

**Courbe de dose réponse en présence d'Antagoniste EDA9**

Le milieu de transfection a été aspiré et une solution de 100nM de BG-LUMI4-TB dans du milieu Krebs-glucose a été ajoutée à chaque puits. La plaque a ensuite été incubée pendant 1h à 37°C en présence de $CO_2$.

Chaque puits a ensuite été lavé 4 fois avec 100 µl de Krebs glucose avant l'ajout de 45 µl de tampon Krebs-glucose ou d'une solution d'EDA9.

Après 30 minutes d'incubation à +37°C et 5% de $CO_2$, 45 µl d'une solution de vasopressine à 160 nM ont été ajoutés dans les puits pour obtenir une concentration finale de vasopressine de 80nM dans chaque puits.

Après 30 minutes d'incubation à +37°C et 5% de $CO_2$ 10µl de fluorescéine en solution à 240 µM dans du tampon Krebs glucose ont été ajoutés dans les puits pour obtenir une concentration finale d'accepteur de 24 µM.

La plaque a ensuite été agitée légèrement puis lue sur un lecteur Envision (Perkin Elmer), équipé d'un laser azote 337 nm et avec des filtres d'émission à 620 et 520 nm. La lecture a été effectuée pour le canal accepteur (520 nm) avec un délai de 60 µs et une fenêtre de lecture de 60 à 400 µs et pour le canal donneur (620 nm) avec un délai de 400 µs pour une fenêtre de 400 à 1500 µs.

**<u>Résultats & Discussion</u>**

La figure 5 représente l'évolution du ratio du signal du donneur (mesuré à 620 nm) sur celui de l'accepteur (mesuré à 520 nm), en fonction de la concentration en antagoniste EDA9 et en présence d'une concentration constante de vasopressine (AVP). Le ratio calculé est multiplié par 10 000 pour en faciliter la représentation graphique.

La figure 5 montre une diminution du ratio lorsque la concentration en antagoniste EDA9 augmente. Cette diminution peut s'expliquer par une diminution du signal du donneur : en l'absence d'EDA9 et en présence de vasopressine, les récepteurs V2 sont internalisés, le donneur n'est pas impliqué dans du DEFET avec l'accepteur présent dans le milieu extracellulaire. En présence d'antagoniste EDA9 qui rentre en compétition avec la vasopressine pour la liaison au récepteur, les récepteurs V2 sont moins activés, donc moins internalisés, le donneur est donc impliqué dans du DEFET avec l'accepteur en solution et sa luminescence est en conséquence moins importante qu'en l'absence d'EDA9.

Cet exemple montre donc que la méthode selon l'invention, en permettant de détecter l'internalisation d'un récepteur, peut permettre de mettre en évidence non seulement des composés agonistes de ce récepteur mais également des composés antagonistes.

**Exemple 4: Détection de l'internalisation du récepteur des chémokines CXCR7**

Cet exemple concerne la détection de l'internalisation du récepteur CXCR7 provoquée par l'action d'un de ses agonistes, le SDF-1 (acronyme du nom anglais de cette molécule, le « Stromal Cell-derived factor 1 », qui signifie facteur 1 dérivé des cellules souches). Le protocole de mise en oeuvre de cet exemple est similaire à celui de l'exemple 2.

Les réactifs et modes opératoires qui ont été utilisés sont les mêmes que ceux de l'exemple 2, à l'exception du plasmide FLAG-ST-V2 qui a été remplacé par le plasmide FLAG-ST-CXCR7 (SEQ ID N° 6), et de la vasopressine qui est remplacée par SDF-1 (Laboratoire Pasteur, Paris).

**Résultats & Discussion**

La figure 6 représente l'évolution du ratio du signal du donneur (mesuré à 620nm) sur celui de l'accepteur (mesuré à 520nm), en fonction de la concentration en SDF-1. Ce ratio permet de s'affranchir des variations inter-puits. Par ailleurs, il a été multiplié par 10 000 pour en faciliter la représentation graphique.

La figure 6 montre très clairement qu'une concentration croissante de SDF-1 provoque une augmentation du ratio, traduisant une augmentation de la luminescence du composé donneur.

Cet exemple montre donc l'excellente corrélation entre l'augmentation du signal du composé donneur correspondant à une baisse de DEFET et l'internalisation du récepteur CXCR7 en présence d'agoniste SDF-1. Cet exemple soutient également le fait que la méthode selon l'invention soit généralisable à tous les récepteurs dont l'activation entraîne l'internalisation.

**Exemple 5: Détection de l'internalisation du récepteur β2-adrénergique**

Cet exemple est similaire à l'exemple 2 mais s'intéresse à la détection de l'internalisation du récepteur β2-adrénergique provoquée par l'action d'un de ses agonistes, l'isoprotérénol.

Les réactifs et modes opératoires qui ont été utilisés sont les mêmes que ceux de l'exemple 2, à l'exception du plasmide FLAG-ST-V2 qui a été remplacé par le plasmide FLAG-ST-β2AR (SEQ ID N°7), et de la vasopressine qui est remplacée par l'isoprotérénol (Tocris).

**Résultats & Discussion**

La figure 7 représente l'évolution du ratio du signal du donneur (mesuré à 620nm) sur celui de l'accepteur (mesuré à 520nm), en fonction de la concentration en isoprotérénol. Ce ratio permet de s'affranchir des variations inter-puits. Par ailleurs, il a été multiplié par 10 000 pour en faciliter la représentation graphique.

La figure 7 montre très clairement qu'une concentration croissante d'isoprotérénol provoque une augmentation du ratio, traduisant une augmentation de la luminescence du composé donneur.

Cet exemple montre donc l'excellente corrélation entre l'augmentation du signal du composé donneur correspondant à une baisse de DEFET et l'internalisation du récepteur β2-adrénergique en présence d'isoprotérénol. Cet exemple soutient également le fait que la méthode selon l'invention soit généralisable à tous les récepteurs dont l'activation entraîne l'internalisation.

**Revendications**

1. Méthode de détection de l'internalisation d'une protéine transmembranaire d'intérêt exprimée à la surface d'une cellule comprenant les étapes suivantes :

a) marquage de la protéine d'intérêt par un complexe métallique fluorescent comprenant un lanthanide ou du

ruthénium, dont la durée de vie est supérieure à 0,1 ms;

b) ajout dans le milieu réactionnel d'un composé susceptible de provoquer l'internalisation de la protéine d'intérêt ;

c) ajout dans le milieu réactionnel d'un agent modulateur choisi parmi :

1. un composé accepteur de FRET fluorescent ou non-fluorescent, compatible avec ledit complexe métallique fluorescent, dont la concentration finale dans le milieu réactionnel est supérieure à $10^{-7}$ M, et dont le poids moléculaire est inférieur à 50 kD;
2. un agent réducteur dont le potentiel redox est compris entre +0,1 V et + 1,2 V;
3. un agent se liant de manière spécifique par liaison non covalente avec le complexe métallique fluorescent ;
4. un ion métallique qui rentre en compétition avec le lanthanide ou le ruthénium pour former un complexe métallique non-fluorescent ;

d) mesure de la luminescence émise par le milieu réactionnel à la longueur d'onde d'émission du complexe métallique fluorescent et/ou à la longueur d'onde d'émission de l'agent modulateur lorsque celui-ci est un composé accepteur fluorescent ;

e) comparaison du signal mesuré à l'étape d) avec un signal de référence mesuré sur des cellules ayant subi uniquement les étapes a) et c), une différence du signal mesuré à l'étape d) par rapport au signal de référence étant représentative de l'internalisation de la protéine d'intérêt.

**2.** Méthode selon la revendication 1, **caractérisée en ce que** les étapes sont mise en oeuvre dans l'ordre suivant : a), b), c), d), e).

**3.** Méthode selon la revendication 1, **caractérisée en ce que** l'agent modulateur est soit un composé accepteur de FRET soit un agent réducteur et **en ce que** les étapes sont mise en oeuvre dans l'ordre suivant : a), c), b), d), e).

**4.** Méthode selon l'une quelconque des revendications 1 à 3, **caractérisée en ce qu'**elle comprend une étape de lavage entre l'étape a) et l'étape suivante.

**5.** Méthode selon l'une quelconque des revendications 1 à 4, **caractérisée en ce que** la durée de vie du complexe métallique fluorescent est comprise entre 0,5 et 6 ms.

**6.** Méthode selon l'une quelconque des revendications 1 à 5, **caractérisée en ce que** le complexe métallique fluorescent est :

a) un complexe de lanthanide dans lequel le lanthanide est choisi parmi l'europium, le terbium, le néodymium, le samarium et le dysprosium ; ou

b) un complexe de ruthénium.

**7.** Méthode selon la revendication 6, **caractérisée en ce que** le complexe métallique fluorescent est un chélate ou un cryptate de lanthanide.

**8.** Méthode selon la revendication 7, **caractérisée en ce que** le complexe métallique fluorescent comprend entre (i) 2 et 9 hétéroatomes donneurs d'électrons choisis parmi : N, O, S, ces atomes formant des liaisons de coordination avec le lanthanide ou le ruthénium, et (ii) un ou plusieurs chromophores comprenant des structures aromatiques, comprenant 1, 2 ou 3 hétéroatomes choisis parmi N et O, qui jouent le rôle d'atomes de coordination du lanthanide ou du ruthénium.

**9.** Méthode selon l'une quelconque des revendications 1 à 8, **caractérisée en ce que** l'agent modulateur est un composé accepteur de FRET fluorescent ou non-fluorescent, compatible avec le complexe métallique fluorescent, dont la concentration finale dans le milieu réactionnel est supérieure à $10^{-7}$ M et dont le poids moléculaire est inférieur à 50 kD.

**10.** Méthode selon la revendication 9, **caractérisée en ce que** la concentration finale du composé accepteur de FRET dans le milieu réactionnel est comprise entre $10^{-6}$ M et $10^{-3}$ M, et **en ce que** son poids moléculaire est compris entre 0,1 et 10 kD.

**11.** Méthode selon l'une quelconque des revendications 1 à 10, **caractérisée en ce que** le lanthanide est le terbium

ou l'europium et l'étape c) consiste à ajouter dans le milieu réactionnel un composé accepteur de FRET fluorescent choisi parmi les composés suivants : les dérivés de cyanines, DY647, D2, CY5, Cy5- COOH, la fluorescéine, la coumarine, la rhodamine, la carbopyronine, l'oxazine ses analogues, les flurophores AlexaFluor, le Crystal violet, les fluorophores perylènebisimide, les fluorophores squaraines, les dérivés du boron- dipyrrométhène, le NBD (nitrobenzoxadiazole) et ses dérivés, le DABCYL (acide 4- ((4- (dimethylamino) phényl) azo) benzoique) .

**12.** Méthode selon l'une quelconque des revendications 1 à 11, **caractérisée en ce que** le composé accepteur de FRET fluorescent est choisi parmi les composés suivants : la fluorescéine et ses dérivés, les dérivés de cyanine.

**13.** Méthode selon l'une quelconque des revendications 1 à 10, **caractérisée en ce que** l'étape c) consiste à ajouter dans le milieu réactionnel un composé accepteur de FRET non-fluorescent choisi parmi les composés suivants : QXL 570, QXL 610, QXL 670 et QXL 680 ; DYQ660 et DYQ661 ; QSY7, QSY9 et QSY21.

**14.** Méthode selon l'une quelconque des revendications 1 à 8, **caractérisée en ce que** l'étape c) consiste à ajouter dans le milieu réactionnel un agent réducteur dont le potentiel redox est compris entre +0,1 V et +1,2 V, choisi parmi les composés suivants : iodure, ascorbate, urate, catécholate, et les espèces inorganiques, telles que le $Fe(CN)_6^{2+}$.

**15.** Méthode selon la revendication 14, **caractérisée en ce que** l'agent modulateur est un agent réducteur et **en ce que** l'étape a) comprend l'ajout d'un premier complexe métallique fluorescent et l'ajout d'un second complexe métallique fluorescent, lesdits complexes métalliques fluorescents ayant les mêmes structures chélatantes mais étant complexées à des métaux différents.

**16.** Méthode selon la revendication 15, **caractérisée en ce que** le premier complexe métallique est un complexe d'europium et le second complexe métallique est un complexe de terbium, les complexes de terbium et d'europium possédant la même structure chélatante.

**17.** Méthode selon l'une des revendications 1 ou 2, **caractérisée en ce que** l'agent modulateur est un agent se liant de manière spécifique par liaison non covalente sur le complexe métallique fluorescent, choisi parmi les composés suivants : anticorps, fragments d'anticorps, peptides, aptamères, chacun possédant un domaine de liaison au complexe métallique fluorescent.

**18.** Méthode selon l'une des revendications 1 ou 2, **caractérisée en ce que** l'agent modulateur est un ion métallique qui entre en compétition avec le lanthanide ou le ruthénium, pour former un complexe métallique non-fluorescent.

**19.** Méthode selon la revendication 18, **caractérisée en ce que** ledit ion est l'ion manganèse.

**20.** Méthode selon l'une quelconque des revendications 1 à 19, **caractérisée en ce que** le marquage de la protéine d'intérêt par le complexe métallique fluorescent est un marquage indirect via un couple de partenaires de liaison, dont l'un au moins est de nature protéique, l'un des membres du couple étant conjugué de manière covalente au complexe métallique fluorescent, et l'autre membre du couple étant conjugué de manière covalente à la protéine d'intérêt ou est présent de manière native sur la protéine d'intérêt.

**21.** Méthode selon l'une quelconque des revendications 1 à 19, **caractérisée en ce que** le marquage de la protéine d'intérêt par le complexe métallique fluorescent est un marquage direct par liaison covalente.

**22.** Méthode selon la revendication 21, **caractérisée en ce que** le complexe métallique fluorescent est conjugué à un substrat d'une enzyme suicide, et **en ce que** la protéine d'intérêt est exprimée sous forme d'une protéine de fusion dont la partie extracellulaire contient l'enzyme suicide.

**23.** Méthode selon la revendication 22, **caractérisée en ce que** le couple enzyme suicide / substrat d'enzyme suicide est choisi parmi les couples suivants : un mutant de l'alkylguanine-DNA alkyltransférase / benzylguanine ; un mutant de l'alkylguanine-DNA alkyltransférase / benzylcytosine; un mutant d'une déshalogénase / chloroalcane ; protéine transporteur d'acyles / Coenzyme A en présence de phosphopantéthéine transférase.

**24.** Méthode selon la revendication 21, **caractérisée en ce que** le complexe métallique fluorescent comporte un premier groupe réactif susceptible de former une liaison covalente avec un second groupe réactif présent sur la protéine d'intérêt, en particulier avec le groupe amino terminal, le groupe carboxylate terminal, les groupes carboxylates des acides aspartiques et glutamiques, les groupes amines des lysines, les groupes guanidines des arginines, les

groupes thiols des cystéines, les groupes phénols des tyrosines, les cycles indoles des tryptophanes, les groupes thioéthers des méthionines, les groupes imidazoles des histidines.

**25.** Méthode selon la revendication 24, **caractérisée en ce que** le complexe métallique fluorescent comporte un groupe maléimide destiné à réagir avec les groupes thiols de la protéine d'intérêt.

**26.** Méthode selon l'une quelconque des revendications 1 à 19, **caractérisée en ce que** le complexe métallique fluorescent est un cryptate d'europium ou de terbium lié de manière covalente à la benzylguanine ou la benzylcytosine ou un de leurs dérivés, et **en ce que** la protéine d'intérêt est exprimée sous forme de protéine de fusion avec un mutant de l'alkylguanine-DNA alkyltransférase.

**27.** Méthode selon l'une quelconque des revendications 1 à 19, caractérisée en que le complexe métallique fluorescent est un cryptate d'europium ou de terbium lié de manière covalente à la benzylguanine ou la benzylcytosine, en ce que la protéine d'intérêt est exprimée sous forme de protéine de fusion avec un mutant de l'alkylguanine-DNA alkyltransférase, et en ce que l'agent modulateur est un dérivé de cyanine, en particulier la Cy5 ou la Cy5-COOH.

**28.** Méthode selon l'une quelconque des revendications 1 à 19, caractérisée en que le complexe métallique fluorescent est un cryptate de terbium lié de manière covalente à la benzylguanine ou la benzylcytosine, en ce que la protéine d'intérêt est exprimée sous forme de protéine de fusion avec un mutant de l'alkylguanine-DNA alkyltransférase, et en ce que l'agent modulateur est la fluorescéine ou un de ses dérivés.

**29.** Méthode selon l'une quelconque des revendications 1 à 28, **caractérisée en ce que** la protéine d'intérêt est choisie parmi : les récepteurs couplés aux protéines G et les récepteurs à activité tyrosine kinase.

**30.** Méthode selon l'une quelconque des revendications 1 à 29, **caractérisée en ce qu'**elle comprend une étape préliminaire de transfection des cellules par un vecteur d'expression comprenant la séquence d'ADN codant pour la protéine d'intérêt, et **en ce que** le vecteur d'expression comprend la séquence codant pour une protéine de fusion dont la partie N-terminale comprend une enzyme suicide, la partie C-terminale comprend la protéine d'intérêt, et **en ce que** la protéine d'intérêt est un RCPG ou un RTK.

**31.** Méthode selon la revendication 30, **caractérisée en ce que** ladite étape de transfection comprend également la cotransfection d'un vecteur comprenant la séquence d'ADN codant pour la β-arrestine 1.

## Patentansprüche

**1.** Verfahren zum Nachweis der Internalisierung eines Transmembranproteins von Interesse, das an der Oberfläche einer Zelle exprimiert wird, umfassend die folgenden Schritte:

a) Markieren des Proteins von Interesse mittels eines fluoreszierenden Metallkomplexes, der ein Lanthanid oder Ruthenium umfasst, wobei seine Lebensdauer mehr als 0,1 ms beträgt,
b) Zusetzen einer Verbindung, welche die Internalisierung des Proteins von Interesse bewirken kann, zum Reaktionsmilieu,
c) Zusetzen eines Modulationsmittels zum Reaktionsmilieu, wobei das Mittel aus den folgenden ausgewählt ist:

1. einer fluoreszierenden oder nicht-fluoreszierenden FRET-Akzeptorverbindung, die mit dem fluoreszierenden Metallkomplex verträglich ist, wobei ihre Endkonzentration im Reaktionsmilieu mehr als $10^{-7}$ M beträgt und wobei ihr Molekulargewicht weniger als 50 kD beträgt,
2. einem Reduktionsmittel, dessen Redoxpotenzial im Bereich von +0,1 V bis + 1,2 V liegt,
3. einem Mittel, das auf spezifische Weise eine nicht-kovalente Bindung mit dem fluoreszierenden Metallkomplex eingeht,
4. einem Metall-Ion, das mit dem Lanthanid oder dem Ruthenium in Konkurrenz tritt, um ein nicht-fluoreszierenden Metallkomplex zu bilden,

d) Messen der Lumineszenz, die das Reaktionsmilieu bei der Emissionswellenlänge des fluoreszierenden Metallkomplexes und/oder bei der Emissionswellenlänge des Modulationsmittels abstrahlt, wenn es sich bei diesem um eine fluoreszierende Akzeptorverbindung handelt,
e) Vergleichen des Signals, das in Schritt d) gemessen wurde, mit einem Referenzsignal, das bei Zellen gemessen wurde, welche ausschließlich den Schritten a) und c) ausgesetzt wurden, wobei eine Differenz gegenüber dem Signal, das in Schritt d) gemessen wird, eine Internalisierung des Proteins von Interesse anzeigt.

2. Verfahren nach Anspruch 1, **dadurch gekennzeichnet, dass** die Schritte in der folgenden Reihenfolge durchgeführt werden: a), b), c), d), e).

3. Verfahren nach Anspruch 1, **dadurch gekennzeichnet, dass** es sich bei dem Modulationsmittel entweder um eine FRET-Akzeptorverbindung oder um ein Reduktionsmittel handelt und dadurch, dass die Schritte in der folgenden Reihenfolge durchgeführt werden: a), c), b), d), e).

4. Verfahren nach einem beliebigen der Ansprüche 1 bis 3, **dadurch gekennzeichnet, dass** es zwischen dem Schritt a) und dem nachfolgenden Schritt einen Waschschritt umfasst.

5. Verfahren nach einem beliebigen der Ansprüche 1 bis 4, **dadurch gekennzeichnet, dass** die Lebensdauer des fluoreszierenden Metallkomplexes im Bereich von 0,5 bis 6 ms liegt.

6. Verfahren nach einem beliebigen der Ansprüche 1 bis 5, **dadurch gekennzeichnet, dass** es sich bei dem fluoreszierenden Metallkomplex handelt um:

   a) einen Lanthanidkomplex, wobei das Lanthanid aus Europium, Terbium, Neodym, Samarium und Dysprosium ausgewählt ist, oder
   b) einen Rutheniumkomplex.

7. Verfahren nach Anspruch 6, **dadurch gekennzeichnet, dass** es sich bei dem fluoreszierenden Metallkomplex um ein Lanthanidchelat- oder -cryptat handelt.

8. Verfahren nach Anspruch 7, **dadurch gekennzeichnet, dass** der fluoreszierende Metallkomplex zwischen (i) 2 und 9 elektronenspendende Heteroatome umfasst, die aus den folgenden ausgewählt sind: N, O, S, wobei diese Atome Koordinationsbindungen mit dem Lanthanid oder dem Ruthenium eingehen, sowie (ii) ein oder mehrere Chromophore, die aromatische Strukturen umfassen, welche 1, 2 oder 3 Heteroatome umfassen, die aus N und O ausgewählt sind und als Koordinationsatome des Lanthanids oder des Rutheniums dienen.

9. Verfahren nach einem beliebigen der Ansprüche 1 bis 8, **dadurch gekennzeichnet, dass** es sich bei dem Modulationsmittel um eine fluoreszierende oder nicht-fluoreszierende FRET-Akzeptorverbindung handelt, die mit dem fluoreszierenden Metallkomplex verträglich ist, wobei ihre Endkonzentration im Reaktionsmilieu mehr als $10^{-7}$ M beträgt und wobei ihr Molekulargewicht weniger als 50 kD beträgt.

10. Verfahren nach Anspruch 9, **dadurch gekennzeichnet, dass** die Endkonzentration der FRET-Akzeptorverbindung in dem Reaktionsmilieu im Bereich von $10^{-6}$ M und $10^{-3}$ M liegt, und dadurch, dass ihr Molekulargewicht im Bereich von 0,1 bis 10 kD liegt.

11. Verfahren nach einem beliebigen der Ansprüche 1 bis 10, **dadurch gekennzeichnet, dass** es sich bei dem Lanthanid um Terbium oder Europium handelt und der Schritt c) darin besteht, dem Reaktionsmilieu eine fluoreszierende FRET- Akzeptorverbindung zuzusetzen, die aus den folgenden Verbindungen ausgewählt ist: Derivaten von Cyaninen, DY647, D2, CY5, Cy5- COOH, Fluorescein, Cumarin, Rhodamin, Carbopyronin, Oxazin und dessen Analoga, Fluorophoren des Typs AlexaFluor, Kristallviolett, Perylenbisimid- Fluorophoren, Squarain- Fluorophoren, Derivaten von Bor- Dipyrromethen, NBD (Nitrobenzoxadiazol) und dessen Derivaten, DABCYL (4- ((4- (Dimethylamino) phenyl) azo) benzoesäure) .

12. Verfahren nach einem beliebigen der Ansprüche 1 bis 11, **dadurch gekennzeichnet, dass** die fluoreszierende FRET-Akzeptorverbindung aus den folgenden Verbindungen ausgewählt ist: Fluorescein und dessen Derivaten, den Cyanin-Derivaten.

13. Verfahren nach einem beliebigen der Ansprüche 1 bis 10, **dadurch gekennzeichnet, dass** der Schritt c) darin besteht, dem Reaktionsmilieu eine nicht-fluoreszierende FRET-Akzeptorverbindung zuzusetzen, die aus den folgenden Verbindungen ausgewählt ist: QXL 570, QXL 610, QXL 670 und QXL 680; DYQ660 und DYQ661; QSY7, QSY9 und QSY21.

14. Verfahren nach einem beliebigen der Ansprüche 1 bis 8, **dadurch gekennzeichnet, dass** der Schritt c) darin besteht, dem Reaktionsmilieu ein Reduktionsmittel zuzusetzen, dessen Redoxpotential im Bereich von +0,1 V bis +1,2 V liegt, wobei es aus den folgenden Verbindungen ausgewählt ist: Iodid, Ascorbat, Urat, Catecholat, sowie anorganischen Spezies wie etwa $Fe(CN)_6^{2+}$.

15. Verfahren nach Anspruch 14, **dadurch gekennzeichnet, dass** es sich bei dem Modulationsmittel um ein Reduktionsmittel handelt, und dadurch, dass der Schritt a) den Zusatz eines ersten fluoreszierenden Metallkomplexes und den Zusatz eines zweiten fluoreszierenden Metallkomplexes umfasst, wobei die fluoreszierenden Metallkomplexe die gleichen chelatbildenden Strukturen haben, aber mit unterschiedlichen Metallen Komplexe bilden.

16. Verfahren nach Anspruch 15, **dadurch gekennzeichnet, dass** es sich bei dem ersten Metallkomplex um einen Europiumkomplex und bei dem zweiten Metallkomplex um einen Terbiumkomplex handelt, wobei die Terbium- und Europiumkomplexe die gleiche chelatbildende Struktur besitzen.

17. Verfahren nach einem der Ansprüche 1 oder 2, **dadurch gekennzeichnet, dass** es sich bei dem Modulationsmittel um ein Mittel handelt, das auf spezifische Weise eine nicht-kovalenten Bindung mit dem fluoreszierenden Metallkomplex eingeht, wobei es aus den folgenden Verbindungen ausgewählt ist: Antikörper, Antikörperfragmente, Peptide, Aptamere, wobei diese jeweils eine Domäne zur Bindung an den fluoreszierenden Metallkomplex besitzen.

18. Verfahren nach einem der Ansprüche 1 oder 2, **dadurch gekennzeichnet, dass** es sich bei dem Modulationsmittel um ein Metall-Ion handelt, das mit dem Lanthanid oder dem Ruthenium in Konkurrenz tritt, um ein nicht-fluoreszierenden Metallkomplex zu bilden.

19. Verfahren nach Anspruch 18, **dadurch gekennzeichnet, dass** es sich bei dem Ion um das Mangan-Ion handelt.

20. Verfahren nach einem beliebigen der Ansprüche 1 bis 19, **dadurch gekennzeichnet, dass** das Protein von Interesse mit dem fluoreszierenden Metallkomplex markiert wird, indem eine indirekte Markierung über ein Paar von Bindungspartnern durchgeführt wird, von denen mindestens einer von proteinartiger Beschaffenheit ist, wobei eines der Mitglieder des Paares auf kovalente Weise mit dem fluoreszierenden Metallkomplex konjugiert ist und wobei das andere Mitglied des Paares auf kovalente Weise mit dem Protein von Interesse konjugiert ist oder auf native Weise an dem Protein von Interesse vorliegt.

21. Verfahren nach einem beliebigen der Ansprüche 1 bis 19, **dadurch gekennzeichnet, dass** das Protein von Interesse mit dem fluoreszierenden Metallkomplex markiert wird, indem eine direkte Markierung mittels einer kovalenten Bindung durchgeführt wird.

22. Verfahren nach Anspruch 21, **dadurch gekennzeichnet, dass** der fluoreszierende Metallkomplex mit dem Substrat eines Suizidenzyms konjugiert ist, und dadurch, dass das Protein von Interesse in Form eines Fusionsproteins exprimiert wird, dessen extrazellulärer Abschnitt das Suizidenzym enthält.

23. Verfahren nach Anspruch 22, **dadurch gekennzeichnet, dass** das Paar Suizidenzym / Suizidenzymsubstrat aus den folgenden Paaren ausgewählt ist: einem Mutanten von Alkylguanin-DNA-Alkyltransferase / Benzylguanin; einem Mutanten von Alkylguanin-DNA-Alkyltransferase / Benzylcytosin; einem Mutanten von Deshalogenase / Chloralkan; Acylgruppen-Transportprotein / Coenzym A in Gegenwart von Phosphopantethein-Transferase.

24. Verfahren nach Anspruch 21, **dadurch gekennzeichnet, dass** der fluoreszierende Metallkomplex eine erste reaktionsfähige Gruppe aufweist, die mit einer zweiten reaktionsfähigen Gruppe eine kovalente Bindung eingehen kann, wobei die letztere Gruppe an dem Protein von Interesse vorliegt und es sich dabei insbesondere um die endständige Aminogruppe, die endständige Carboxylatgruppe, die Carboxylatgruppen von Asparaginsäure und Glutaminsäure, die Amingruppen der Lysine, die Guanidingruppen der Arginine, die Thiolgruppen der Cysteine, die Phenolgruppen der Tyrosine, die Indolringe der Tryptophane, die Thioethergruppen der Methionine, die Imidazolgruppen der Histidine handelt.

25. Verfahren nach Anspruch 24, **dadurch gekennzeichnet, dass** der fluoreszierende Metallkomplex eine Maleimidgruppe aufweist, die dazu bestimmt ist, mit den Thiolgruppen des Proteins von Interesse zu reagieren.

26. Verfahren nach einem beliebigen der Ansprüche 1 bis 19, **dadurch gekennzeichnet, dass** es sich bei dem fluoreszierenden Metallkomplex um ein Europium- oder Terbiumcryptat handelt, welches auf kovalente Weise an Benzylguanin oder Benzylcytosin oder eines ihrer Derivate gebunden ist, und dadurch, dass das Protein von Interesse in Form eines Fusionsproteins mit einem Mutanten der Alkylguanin-DNA-Alkyltransferase exprimiert wird.

**27.** Verfahren nach einem beliebigen der Ansprüche 1 bis 19, **dadurch gekennzeichnet, dass** es sich bei dem fluoreszierenden Metallkomplex um ein Europium- oder Terbiumcryptat handelt, welches auf kovalente Weise an Benzylguanin oder Benzylcytosin gebunden ist, und dadurch, dass das Protein von Interesse in Form eines Fusionsproteins mit einem Mutanten der Alkylguanin-DNA-Alkyltransferase exprimiert wird, und dadurch, dass es sich bei dem Modulationsmittel um ein Cyanin-Derivat handelt, insbesondere um Cy5 oder Cy5-COOH.

**28.** Verfahren nach einem beliebigen der Ansprüche 1 bis 19, **dadurch gekennzeichnet, dass** es sich bei dem fluoreszierenden Metallkomplex um ein Terbiumcryptat handelt, welches auf kovalente Weise an Benzylguanin oder Benzylcytosin gebunden ist, und dadurch, dass das Protein von Interesse in Form eines Fusionsproteins mit einem Mutanten der Alkylguanin-DNA-Alkyltransferase exprimiert wird, und dadurch, dass es sich bei dem Modulationsmittel um Fluorescein oder eines seiner Derivate handelt.

**29.** Verfahren nach einem beliebigen der Ansprüche 1 bis 28, **dadurch gekennzeichnet, dass** das Protein von Interesse aus den folgenden ausgewählt ist: den Rezeptoren, die an das G-Protein gekoppelt sind, und den Rezeptoren mit Tyrosinkinase-Aktivität.

**30.** Verfahren nach einem beliebigen der Ansprüche 1 bis 29, **dadurch gekennzeichnet, dass** es im Vorfeld einen Schritt der Transfektion der Zellen mittels eines Expressionsvektors umfasst, welcher die DNA-Sequenz umfasst, die für das Protein von Interesse codiert, und dadurch, dass der Expressionsvektor die Sequenz umfasst, die für ein Fusionsprotein codiert, dessen N-terminaler Abschnitt ein Suizidenzym umfasst, wobei der C-terminale Abschnitt das Protein von Interesse umfasst, und dadurch, dass es sich bei dem Protein von Interesse um ein RCPG oder ein RTK handelt.

**31.** Verfahren nach Anspruch 30, **dadurch gekennzeichnet, dass** der Transfektionsschritt weiterhin die Cotransfektion eines Vektors umfasst, welcher die DNA-Sequenz umfasst, die für β-Arrestin 1 codiert.

## Claims

**1.** A method for detecting the internalization of a transmembrane protein of interest expressed at the surface of a cell, comprising the following steps:

a) labeling the protein of interest with a fluorescent metal complex comprising a lanthanide or ruthenium, the lifetime of which is greater than 0.1 ms;
b) adding to the reaction medium a compound capable of causing the internalization of the protein of interest;
c) adding to the reaction medium a modulating agent selected from:

1. a fluorescent or nonfluorescent FRET acceptor compound compatible with said fluorescent metal complex, the final concentration of which in the reaction medium is greater than $10^{-7}$ M, and the molecular weight of which is less than 50 kD;
2. a reducing agent, the redox potential of which is between +0.1 V and +1.2 V;
3. an agent which binds specifically, by noncovalent bonding, with the fluorescent metal complex;
4. a metal ion which competes with the lanthanide or the ruthenium so as to form a nonfluorescent metal complex;

d) measuring the luminescence emitted by the reaction medium at the emission wavelength of the fluorescent metal complex and/or at the emission wavelength of the modulating compound when said compound is a fluorescent acceptor compound;
e) comparing the signal measured in step d) with a reference signal measured on cells having been subjected only to steps a) and c), a difference in the signal measured in step d) compared with the reference signal being representative of the internalization of the protein of interest.

**2.** The method as claimed in claim 1, **characterized in that** the steps are carried out in the following order: a), b), c), d), e).

**3.** The method as claimed in claim 1, **characterized in that** the modulating agent is either a FRET acceptor compound

or a reducing agent, and **in that** the steps are carried out in the following order: a), c), b), d), e).

4. The method as claimed in any one of claims 1 to 3, **characterized in that** it comprises a washing step between step a) and the following step.

5. The method as claimed in any one of claims 1 to 4, **characterized in that** the lifetime of the fluorescent metal complex is between 0.5 and 6 ms.

6. The method as claimed in any one of claims 1 to 5, **characterized in that** the fluorescent metal complex is:

   a) a lanthanide complex in which the lanthanide is selected from europium, terbium, neodymium, samarium and dysprosium; or
   b) a ruthenium complex.

7. The method as claimed in claim 6, **characterized in that** the fluorescent metal complex is a lanthanide chelate or cryptate.

8. The method as claimed in claim 7, **characterized in that** the fluorescent metal complex comprises (i) between 2 and 9 electron-donor heteroatoms selected from N, O and S, these atoms forming coordination bonds with the lanthanide or the ruthenium, and (ii) one or more chromophores comprising aromatic structures, comprising 1, 2 or 3 heteroatoms selected from N and O, which play the role of lanthanide or ruthenium coordination atoms.

9. The method as claimed in any one of claims 1 to 8, **characterized in that** the modulating agent is a fluorescent or nonfluorescent FRET acceptor compound compatible with the fluorescent metal complex, the final concentration of which in the reaction medium is greater than $10^{-7}$ M and the molecular weight of which is less than 50 kD.

10. The method as claimed in claim 9, **characterized in that** the final concentration of the FRET acceptor compound in the reaction medium is between $10^{-6}$ M and $10^{-3}$ M, and **in that** its molecular weight is between 0.1 and 10 kD.

11. The method as claimed in any one of claims 1 to 10, **characterized in that** the lanthanide is terbium or europium, and **in that** step c) comprises adding to the reaction medium a fluorescent FRET acceptor compound selected from the following compounds: cyanin derivatives, DY647, D2, CY5, Cy5- COOH, fluorescein, coumarin, rhodamine, carbopyronine, oxazine and its analogs, AlexaFluor fluorophores, Crystal violet, perylene bisimide fluorophores, squaraine fluorophores, boron dipyrromethene derivatives, NBD (nitrobenzoxadiazole) and its derivatives, DABCYL (4- ((4- (dimethylamino) phenyl) azo) benzoic acid) .

12. The method as claimed in any one of claims 1 to 11, **characterized in that** the fluorescent FRET acceptor compound is selected from the following compounds: fluorescein and its derivatives, and cyanin derivatives.

13. The method as claimed in any one of claims 1 to 10, **characterized in that** step c) comprises adding to the reaction medium a non-fluorescent FRET acceptor compound selected from the following compounds: QXL 570, QXL 610, QXL 670 and QXL 680; DYQ660 and DYQ661; QSY7, QSY9 and QSY21.

14. The method as claimed in any one of claims 1 to 8, **characterized in that** step c) comprises adding to the reaction medium a reducing agent the redox potential of which is between +0.1 V and +1.2 V, selected from the following compounds: iodide, ascorbate, urate, catecholate, and inorganic species, such as $Fe(CN)_6^{2+}$.

15. The method as claimed in claim 14, **characterized in that** the modulating agent is a reducing agent and **in that** step a) comprises adding a first fluorescent metal complex and adding a second fluorescent metal complex, said fluorescent metal complexes having the same chelating structures but being complexed with different metals.

16. The method as claimed in claim 15, **characterized in that** the first metal complex is a europium complex and the second metal complex is a terbium complex, the terbium and europium complexes having the same chelating structure.

17. The method as claimed in either of claims 1 or 2, **characterized in that** the modulating agent is an agent which binds specifically, by noncovalent bonding, to the fluorescent metal complex, selected from the following compounds: antibodies, antibody fragments, peptides or aptamers, each having a fluorescent- metal- complex- binding domain.

**18.** The method as claimed in either of claims 1 and 2, **characterized in that** the modulating agent is a metal ion which competes with the lanthanide or the ruthenium, so as to form a nonfluorescent metal complex.

**19.** The method as claimed in claim 18, **characterized in that** said ion is the manganese ion.

**20.** The method as claimed in any one of claims 1 to 19, **characterized in that** the labeling of the protein of interest with the fluorescent metal complex is indirect labeling via a pair of binding partners, at least one of which is protein in nature, one of the members of the pair is covalently conjugated to the fluorescent metal complex, and the other member of the pair is covalently conjugated to the protein of interest or is naturally present on the protein of interest.

**21.** The method as claimed in any one of claims 1 to 19, **characterized in that** the labeling of the protein of interest with the fluorescent metal complex is direct labeling by covalent bonding.

**22.** The method as claimed in claim 21, **characterized in that** the fluorescent metal complex is conjugated to a substrate of a suicide enzyme, and **in that** the protein of interest is expressed in the form of a fusion protein, the extracellular part of which contains the suicide enzyme.

**23.** The method as claimed in claim 22, **characterized in that** the suicide enzyme / suicide enzyme substrate pair is selected from the following pairs: an alkylguanine-DNA alkyltransferase mutant / benzylguanine; an alkylguanine-DNA alkyltransferase mutant / benzylcytosine; a dehalogenase mutant / chloroalkane; an acyl carrier protein / coenzyme A in the presence of phosphopantetheine transferase.

**24.** The method as claimed in claim 21, **characterized in that** the fluorescent metal complex comprises a first reactive group capable of forming a covalent bond with a second reactive group present on the protein of interest, in particular with the terminal amino group, the terminal carboxylate group, the carboxylate groups of aspartic acids and glutamic acids, the amine groups of lysines, the guanidine groups of arginines, the thiol groups of cysteines, the phenol groups of tyrosines, the indole rings of tryptophans, the thioether groups of methionines, the imidazole groups of histidines.

**25.** The method as claimed in claim 24, **characterized in that** the fluorescent metal complex comprises a maleimide group intended to react with the thiol groups of the protein of interest.

**26.** The method as claimed in any one of claims 1 to 19, **characterized in that** the fluorescent metal complex is a europium cryptate or a terbium cryptate covalently bonded to benzylguanine or benzylcytosine or one of their derivatives, and **in that** the protein of interest is expressed in the form of a fusion protein with an alkylguanine-DNA alkyltransferase mutant.

**27.** The method as claimed in any one of claims 1 to 19, **characterized in that** the fluorescent metal complex is a europium cryptate or a terbium cryptate covalently bonded to benzylguanine or benzylcytosine, **in that** the protein of interest is expressed in the form of a fusion protein with an alkylguanine-DNA alkyltransferase mutant, and **in that** the modulating agent is a cyanin derivative, in particular Cy5 or Cy5-COOH.

**28.** The method as claimed in any one of claims 1 to 19, **characterized in that** the fluorescent metal complex is a terbium cryptate covalently bonded to benzylguanine or benzylcytosine, **in that** the protein of interest is expressed in the form of a fusion protein with an alkylguanine-DNA alkyltransferase mutant, and **in that** the modulating agent is fluorescein or one of its derivatives.

**29.** The method as claimed in any one of claims 1 to 28, **characterized in that** the protein of interest is selected from: G-protein coupled receptors and receptors tyrosine kinase.

**30.** The method as claimed in any one of claims 1 to 29, **characterized in that** it comprises a preliminary step of transfecting cells with an expression vector comprising the DNA sequence encoding the protein of interest, and **in that** the expression vector comprises a sequence encoding a fusion protein, the N-terminal part of which comprises a suicide enzyme and the C-terminal part of which comprises the protein of interest, and **in that** the protein of interest is a GPCR or an RTK.

**31.** The method as claimed in claim 30, **characterized in that** said transfection step also comprises co-transfecting a vector comprising the DNA sequence encoding β-arrestin 1.

# FIGURE 1

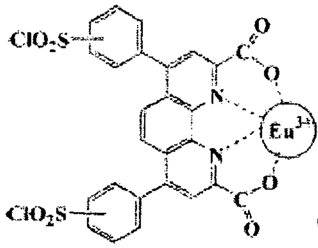
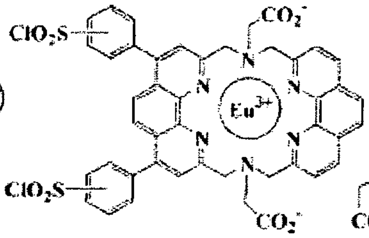
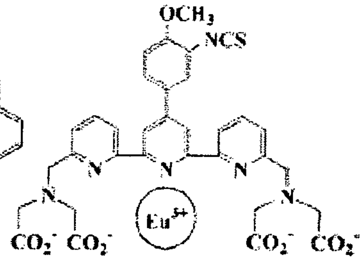
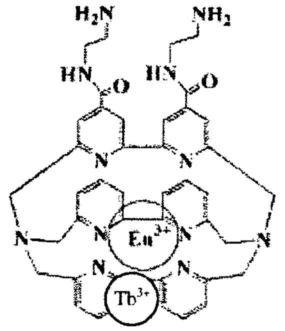
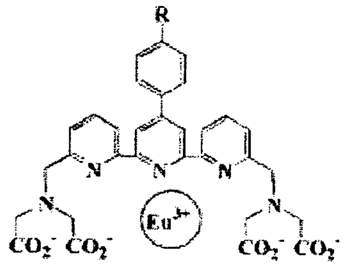
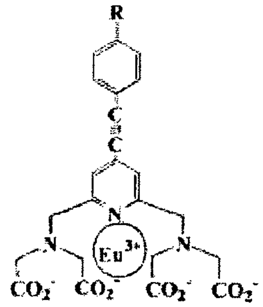
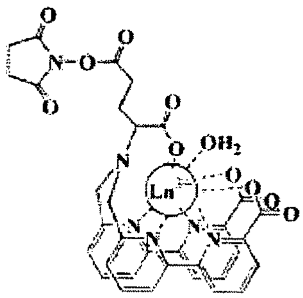
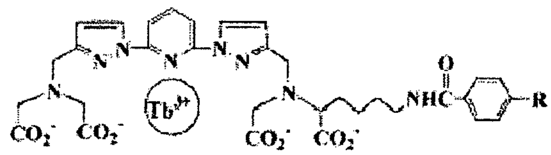

R = -NCS or 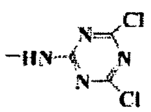

$Ln^{3+} = Eu^{3+}$ or $Tb^{3+}$

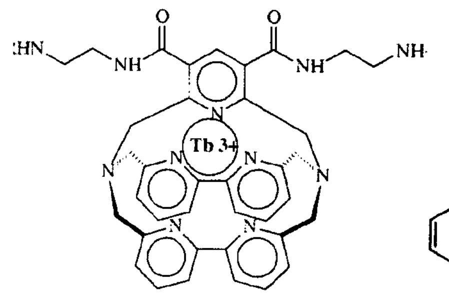
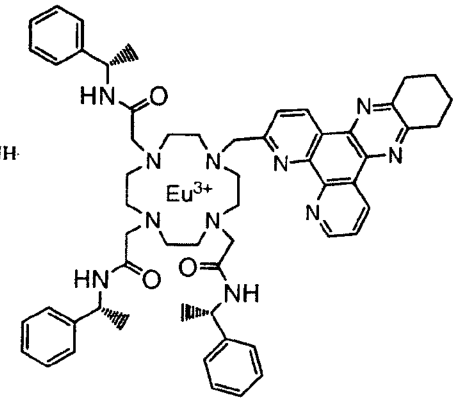
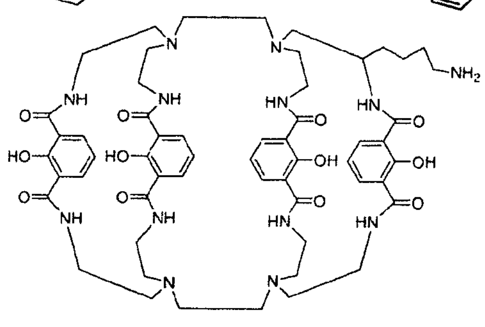

**FIGURE 2**

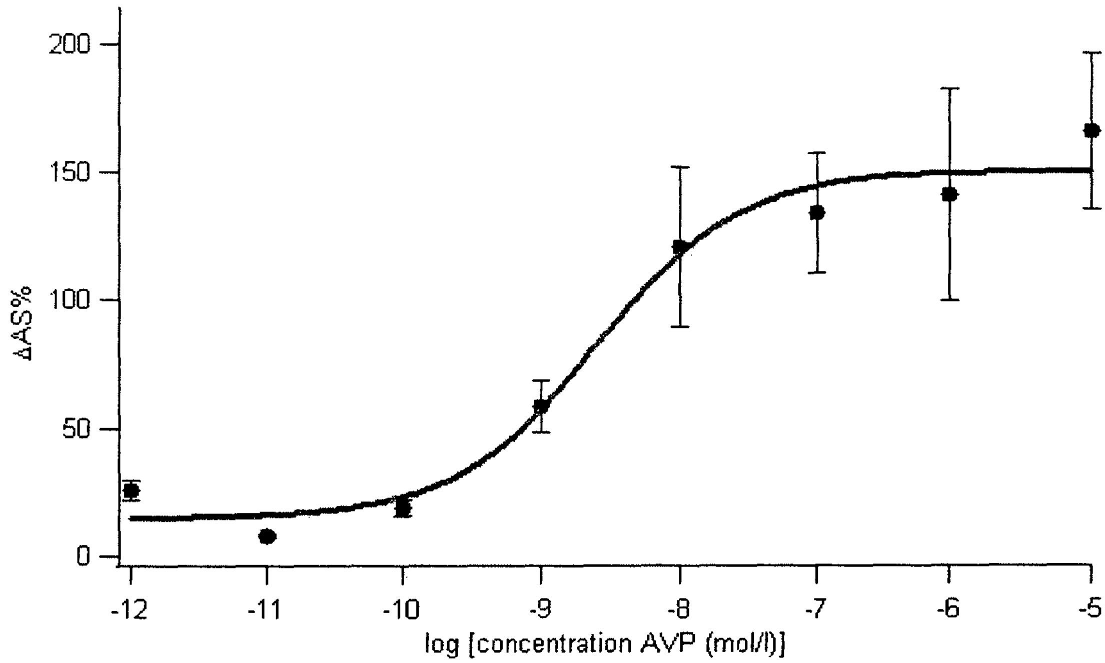


**FIGURE 3**

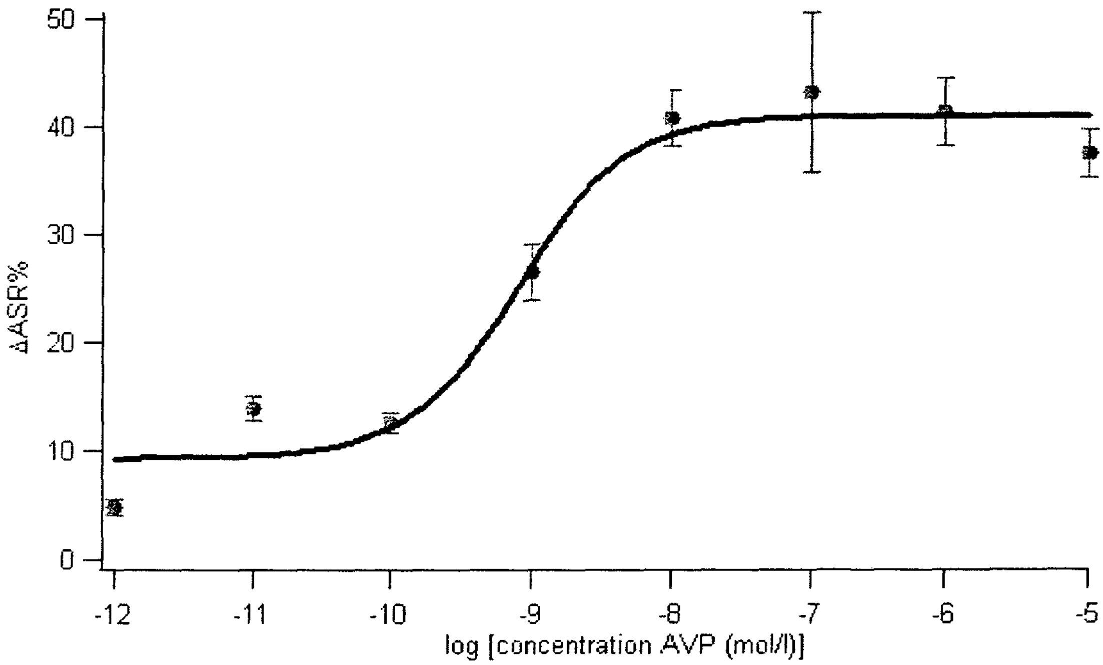

FIGURE 4

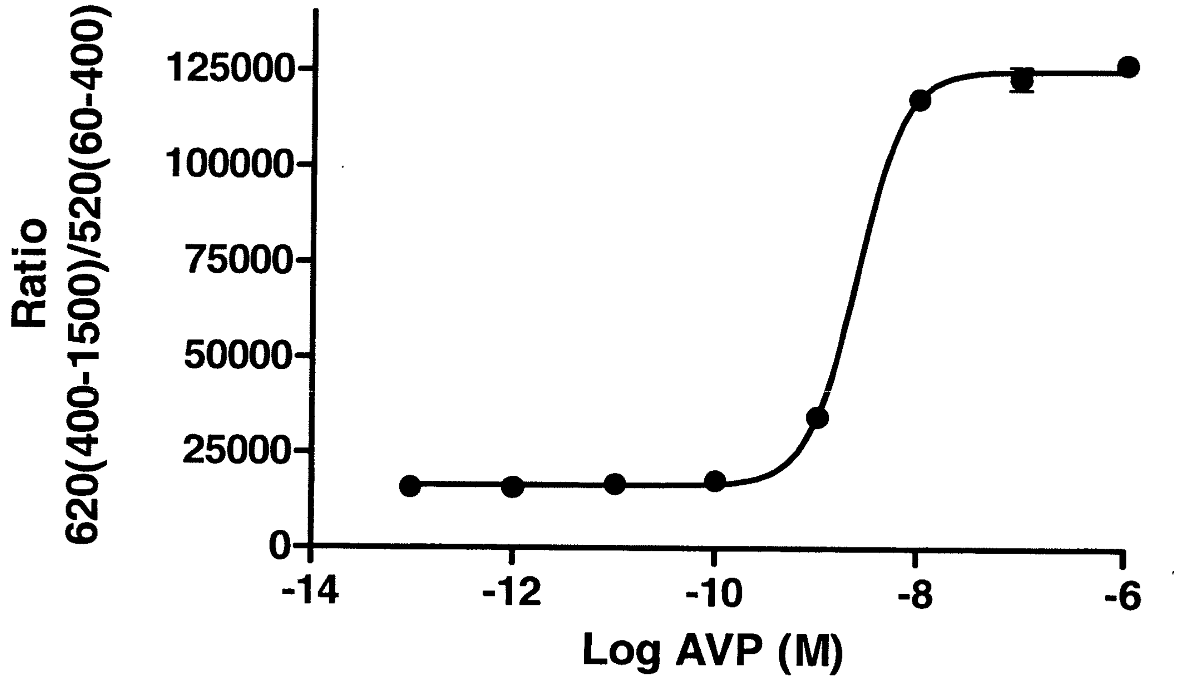


FIGURE 5

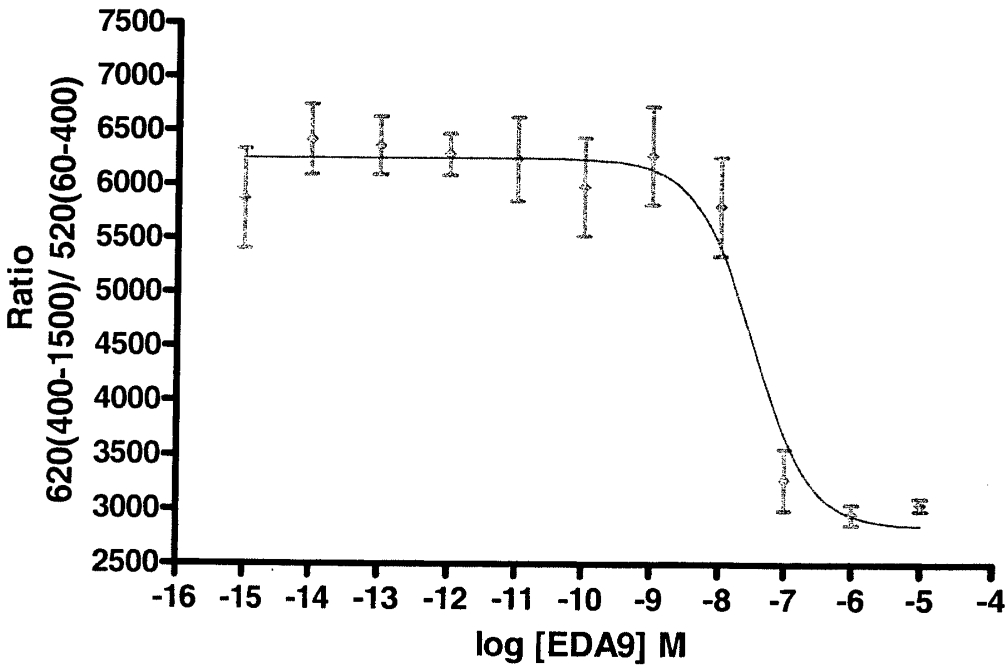

FIGURE 6

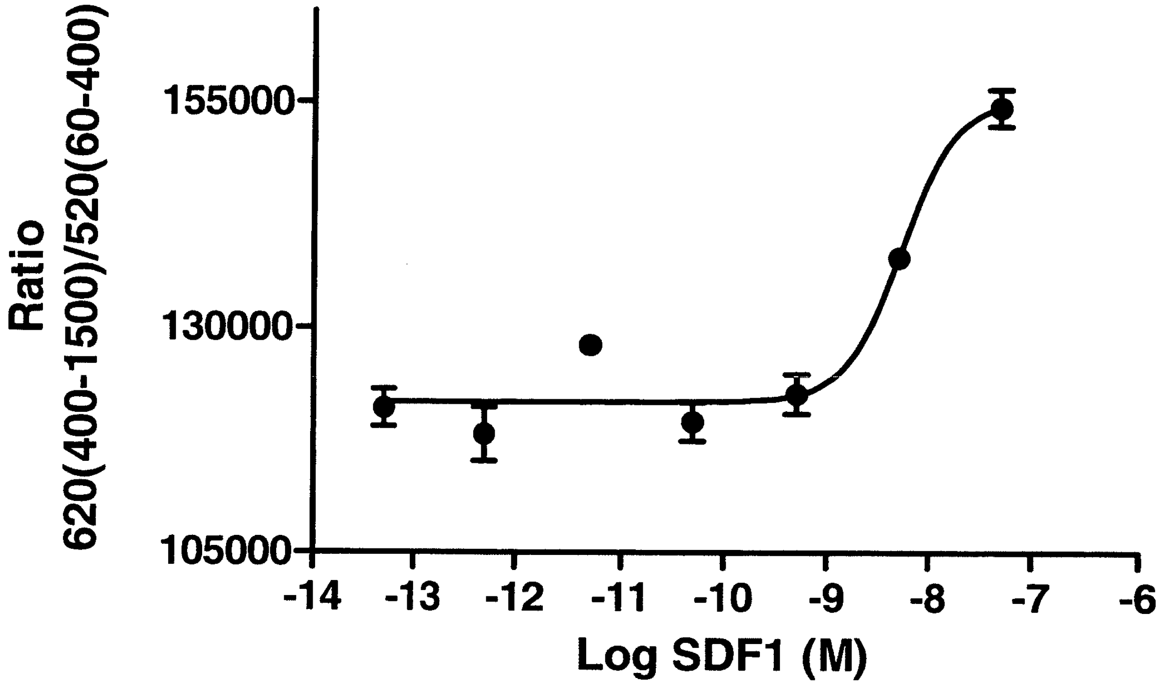


FIGURE 7

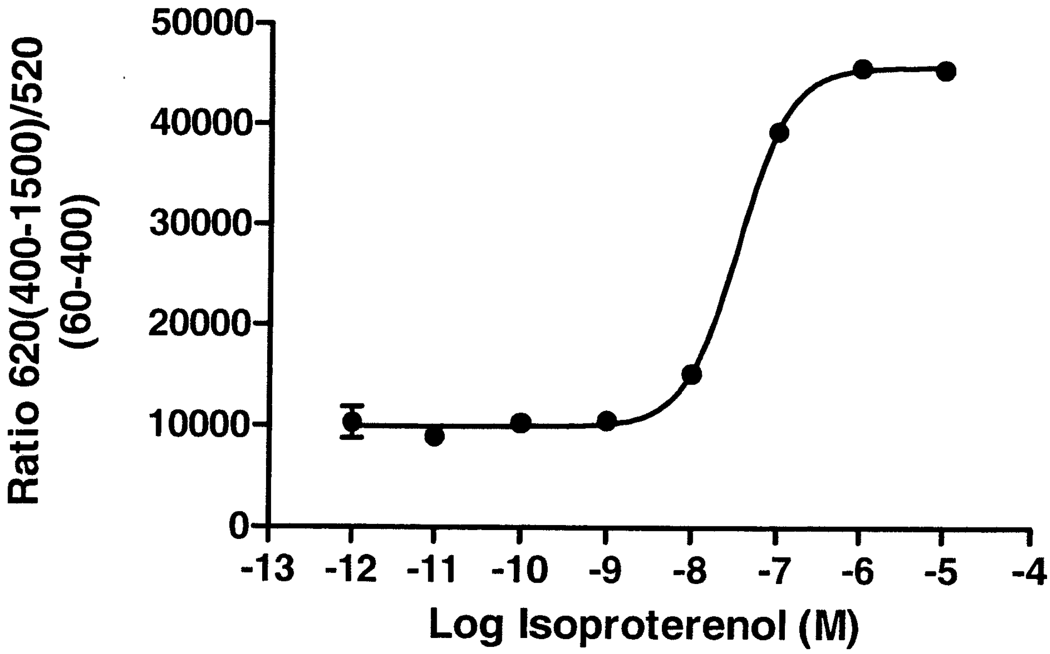

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0167106 A
- WO 0159451 A
- WO 0158923 A
- WO 2005007822 A
- WO 04065963 A
- EP 1015608 A
- WO 0003246 A
- WO 0075237 A
- GB 2223096 A
- WO 2008007089 A
- US 2005026234 A
- WO 2008103393 A
- EP 0180492 A
- EP 0321353 A
- EP 0601113 A
- WO 0196877 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 0403593 A
- US 5324825 A
- US 5202423 A
- US 5316909 A
- WO 200910580 A
- EP 1154991 A
- EP 1154990 A
- WO 2008063721 A
- US 5622821 A
- WO 2007116069 A1
- WO 04072232 A2
- FR 0855296


### Littérature non-brevet citée dans la description


- **GAINETDINOV et al.** *Annu Review Neurosci,* 2004, vol. 27, 107-44
- **CHAREST et al.** *EMBO Rep.,* Avril 2005, vol. 6 (4), 334-40
- **MATHIS et al.** *Clin. Chem.,* Septembre 1995, vol. 41 (9), 1391-7
- **D. THOMAS et al.** *PNAS,* 1978, vol. 75 (12), 5746-5750
- **ZHENG et al.** *J. Am. Chem. Soc.,* 2005, vol. 127, 16178-16188
- *Org. Biomol. Chem.,* 2007, vol. 5, 2975-2982
- **DHONUKSHE et al.** *Current Biology, Current Science,* 2007, vol. 17 (6), 520-527
- **REAVES et al.** *Journal of Cell Science,* 1996, vol. 109 (4), 749-762
- **ROOT.** *Proc. Nat. Acad. Sci. USA,* 1997, vol. 94 (11), 5685-5690
- **MAUREL et al.** *Analytical Biochemistry,* 2004, vol. 329 (2), 253-262
- **POOLE R. et al.** *Biomol. Chem,* 2005, vol. 3, 1013-1024
- **KIELAR et al.** *Org. Biomol. Chem.,* 2007, vol. 5, 2975-2982
- **B.A.GRIFFIN et al.** *Science,* 1998, vol. 281, 269-272
- **S.A. ADAMS et al.** *J. Am. Chem. Soc.,* 2002, vol. 124, 6063-6076
- **C. M. MCCANN et al.** *Biotechnique,* 2005, vol. 38, 945-952
- **JUILLERAT et al.** *Chemistry & biology,* Avril 2003, vol. 10, 313-317
- **GAUTIER et al.** *Chemistry et Biology,* Février 2008, vol. 15, 128-136
- **GRONEMEYER et al.** *Protein engineering, design & selection,* 2006, vol. 19 (7), 309-316
- **N.GEORGE et al.** *Journal of the American Chemical society,* 2004, vol. 126, 8896-8897